# EUROPEAN PATENT SPECIFICATION

(11) **EP 4 572 746 B1**
(45) Date of publication and mention of the grant of the patent: **08.07.2026**
(21) Application number: 23757361.3
(22) Date of filing: 06.08.2023
(51) Int. Cl.: A61K 9/70, A61K 47/26, A61K 9/107, A61K 47/10, A61P 17/12, A61P 31/10

(54) **TOPICAL DELIVERY OF ANTIFUNGAL AGENTS FOR TREATING FUNGAL INFECTIONS**
TOPISCHE VERABREICHUNG VON ANTIFUNGALEN MITTELN ZUR BEHANDLUNG VON PILZINFEKTIONEN
ADMINISTRATION TOPIQUE D'AGENTS ANTIFONGIQUES POUR LE TRAITEMENT D'INFECTIONS FONGIQUES

(30) Priority: 17.08.2022 IL 29572522
(43) Date of publication of application: 25.06.2025
(73) Proprietor: Lyotropic Delivery Systems Ltd., 9139002 Jerusalem (IL)
(72) Inventor: GARTI, Nissim, 4723127 Ramat HaSharon (IL); GARTI-LEVI, Sharon, 7173871 Modi'in (IL); ABU-GHOUSH, Reham, Beit Hanina, Jerusalem (IL); EDRI, Rotem, 8826222 Eilat (IL)
(74) Representative: Vossius & Partner Patentanwälte Rechtsanwälte mbB
(86) International application number: PCT/IL2023/050814
(87) International publication number: WO 2024/038427

(56) References cited:
- EP-A1- 3 284 484
- US-A1- 2008 317 737
- US-A1- 2015 111 971
- VIKAS AGRAWAL ET AL: "Mechanistic Insights of Formulation Approaches for the Treatment of Nail Infection: Conventional and Novel Drug Delivery Approaches", AAPS PHARMSCITECH, vol. 21, no. 2, 2 January 2020 (2020-01-02), XP037400021, DOI: 10.1208/S12249-019-1591-9

## Description

### TECHNOLOGICAL FIELD

The present disclosure concerns formulations for topical delivery of antifungal agents through keratinous tissues, *e.g*. nail and/or skin.

### BACKGROUND ART

References considered to be relevant as background to the presently disclosed subject matter are listed below:
[1] K Foster et al., J Am Acad Dermatol 2004, 50, 748-752
[2] AB Youssef et al., J Mycol Med. 2018, 28(4), 651-654
[3] A Shemer, Dermatol. Ther. 2012, 25(6), 582-593
[4] BM Piraccini et al., Skin Appendage Disord. 2018, 5(1), 13-19
[5] AK Gupta, Expert Rev. Anti Infect. Ther. 2018, 16(12), 929-938
[6] M Johnson et al., British Journal of Dermatology 1994, 130, 195-198
[7] VK Bhatia et al., Indian Journal of Medical Microbiology 2015, 33(4), 533-537
[8] VK Maurya et al., J. Fam. Med. Prim. Care 2019, 8(8), 2577
[9] PCT patent application publication no. WO2020/255114
[10] US2015/111971

Acknowledgement of the above references herein is not to be inferred as meaning that these are in any way relevant to the patentability of the presently disclosed subject matter.

### BACKGROUND

Onychomycosis or *Tinea unguium* is a chronic fungal infection of the nail unit affecting 2-18% of the population worldwide. The observed incidence increases with diminished levels of immune competence (*e.g*. diabetes), age (prevalence rate in children is 0.2%-2.6% vs 48% in age over 70) or trauma to the nail unit [1].

The most frequent cause of onychomycosis is *Trichophyton rubrum,* but other dermophytes including *Trichophyton mentagrophytes* and *Epidermophyton floccosum,* can be caused as well. The dermatophytes are identified in 90% of the toenail and 50% of fingernail onychomycosis. *Candida albicans* accounts for 2% of onychomycosis, occurring especially in fingernails. Non-dermatophytic mold onychomycosis is primarily cultured from toenails. Examples of these saprophytic molds include *Fusarium, Aspergillus, Acremonium scytalidium* and *Scopulariopsis brevicaulis.* They account for about 8% of nail infections [2].

Distal lateral subungual onychomycosis is the most common clinical subtype. In distal lateral subungual onychomycosis, the fungal invasion begins at the hyponychium and then progresses to involve the distal nail bed and subsequently the nail plate [3]. Clinically, distal lateral subungual onychomycosis presents as yellowish, whitish, or brownish discoloration of a distal corner of a nail. Distal subungual hyperkeratosis, onycholysis, and/or onychauxis of the lateral and distal aspects of the nail plate are common [3].

Topical antifungal therapy involves the use of nail and skin lacquers and solutions in addition to topical antifungal agents. Commonly used topical antifungal agents include efinaconazole (Jublia, Clenafin) (10% nail solution), tavaborole (Kerydin) (5% nail solution), ciclopirox (Ciclodan, Penlac, Loprox) (8% nail lacquer or hydrolacquer), amorolfine (Curanail, Loceryl, Locetar, Odenil) (5% nail lacquer), and terbinafine (Lamisil) (1% nail solution) [4]. Generally, topical antifungal agents are well tolerated; adverse events are minimal and include periungual erythema and burning at the application site [5].

Oral antifungal drugs are the gold standard for treating onychomycosis, with mycological and complete cure rates of 70% and 38%, respectively. Systemic agents are only very slightly effective (with an average of 3-7% complete cure rate) with potentially severe adverse effects and possible drug-drug interactions. Treatment failure (20%-50%) and recurrence rates (10%-53%) are high and rising. Topical antifungal agents may be preferred over systemic agents due to lack of systemic adverse exposure.

### GENERAL DESCRIPTION

The present disclosure provides formulations according to the claims for topical delivery of an antifungal agent to a keratinous tissue for prolonged delivery of the antifungal agent to the tissue. The formulations described herein are in the form of nanodomains dispersed in an aqueous phase, having a viscosity suitable for topical application of a thin layer of the formulation to the keratinous tissue, and is formulated to form a film onto the keratinous tissue upon application and the evaporation of the solvents/water, and has a residence time of at least few hours, *e.g*. up to 24 hours. The film that resides on the tissue after application carries the nanodomains that contain the antifungal agent and permits prolonged release of the antifungal agent to the tissue during contact of the film with the tissue. Hence, the formulation, after application, forms a depot of the antifungal agent onto the tissue. Further, the nanodomains, due to their size and composition, permit effective local delivery of the antifungal agent to the keratinous layer, without having significant systemic exposure. It was also surprisingly found, as will be further described below, that formulations of this disclosure permit enhanced permeation and diffusion of the antifungal agent through the keratinous tissue, such that the antifungal agent is delivered not only to the area of the tissue onto which the formulation is applied, but also deep into the keratinous layers and to laterally adjacent tissue area, enabling effective treatment of fungal-infected tissue.

By one of its aspects, the disclosure provides a formulation for topical delivery of an antifungal active agent to a keratinous tissue, the formulation comprising nanodomains dispersed in an aqueous continuous phase. The nanodomains comprise the antifungal active agent, at least one hydrophilic surfactant, at least one alcohol, and at least one polyol, while the aqueous phase comprises water, at least one film-forming agent in an amount of no more than about 1 wt%, and at least one keratolytic agent. The total amount of water in the formulation is between about 35 wt% and about 55 wt%.

The formulations of this disclosure are liquid formulations, having a nanostructure that consists of nanodomains, *i.e*. nanodroplets, that contain and stabilize an antifungal active agent, and an aqueous continuous phase in which the nanodomains are dispersed. As the nanodomains typically have an average size of no more than 100 nm, more typically no more than 50 nm, the formulation is transparent, as the droplets do not disrupt passage of visible light through the formulation.

By some embodiments, the nanodomains have an average size of no more than about 100 nm (nanometers). By other embodiments, the nanodomains have an average size of no more than about 50 nm. According to some embodiments, the nanodomains have an average size of between about 1 and about 25 nm.

According to some embodiments, the formulation is in the form of a homogenous dispersion of nanodomains in an aqueous phase. The formulation is a system in which droplets, typically of nanometric size and structure, are self-assembled in a spontaneous manner, such that the formed nanodomains are stable in the formulation both kinetically and thermodynamically. In other words, no rigorous mixing or shearing forces need to be applied onto the components of the system for arrangement in a form of nanodroplets that are homogenously dispersed in a continuous phase. In the formulations of the present disclosure, the balance between the at least one hydrophilic surfactant, at least one alcohol, and at least one polyol enables obtaining droplets having substantially zero interfacial energy, thereby inducing the components to spontaneously arrange in an energetically favorable nanostructure, which is both kinetically and thermodynamically stable.

According to some embodiments, the formulations are in the form of nanodomains dispersed in a continuous aqueous phase. Contrary to emulsion systems, which are rich in oil so that the active agent is typically dissolved within an oil core, in the formulations of this disclosure the absence of oil forces the antifungal agent to be entrapped within the tails of the surfactants, and hence reside at the interface between the nanodomains and the aqueous phase. Such solubilization within the interface results in highly thermodynamically stable formulation, that does not undergo phase separation, or release of the antifungal agent from the nanodroplets over prolonged periods of time, while only upon contacting the keratinous tissue the active can be released from the formulation.

Thus, by some embodiments, the formulation comprises at most 1wt% of oil. In some other embodiments, the formulation is devoid of oil.

The formulations are designed to deliver an antifungal active agent to a keratinous tissue. The term *antifungal active agent* means to denote one or more chemical entities having an effect of delaying, arresting, diminishing and/or preventing at least one effect associated with fungal contamination in a keratinous tissue. The activity of the antifungal agent can be by way of treatment or prevention. The antifungal active agent referred to herein has at least one effect of ameliorating undesired symptoms associated with a fungal infection, preventing the manifestation of such symptoms before they occur, slowing down the progression of the fungal infection, enhancing the onset of remission period, slowing down irreversible damage caused in progressive stages of the infection, delaying the onset of said progressive stage, lessening the severity or cure the infection, or preventing the infection from reoccurring, or a combination of two or more of the above.

According to some embodiments, the antifungal agent is selected from polyenes (amphotericin B, nystatin, *etc*.), azoles (itraconazole, fluconazole, efinaconazole, ketoconazole, miconazole, *etc*.), allylamines (terbinafine, butenafine, *etc*.) and other synthetic and natural molecules including, but not limited to, ciclopirox, amorolfine, tavaborole, griseofulvin, or any pharmaceutically acceptable salt, hydrate, derivative or analogue thereof.

By some embodiments, the antifungal is terbinafine or any suitable pharmaceutically acceptable salt thereof.

According to some embodiments, the antifungal active agent is present in the formulation in an amount of at least about 1 wt%. By some other embodiments, the antifungal active agent is present in the formulation in an amount ranging between about 1 wt% and about 10 wt%.

The formulations are designed for topical administration to a keratinous tissue. *Keratinous tissue* means to encompass a tissue rich in keratin, such as nails or upper layers of skin surrounding the nail or nail bed.

According to some embodiments, the formulations are for use in treating a fungal infection in human subjects. In such embodiments, the keratinous tissue is at least one of nail, nail bed, eponychium (cuticle), perionychium (skin surrounding the nail), hyponychium (skin under the nail plate), and hair.

According to other embodiments, the formulations are for veterinary use, *e.g.* for use in treating a fungal infection in a mammalian or non-mammalian animal. In such embodiments, the keratinous tissue is at least one of nail, keratinous skin, hair (including fur and wool), horn, hoof, feathers, scales, *etc.*

As noted, the nanodomains comprise at least one hydrophilic surfactant, at least one alcohol, and at least one polyol.

The *hydrophilic surfactant* is a surface-active agent that has a hydrophilic head group and lipophilic tails that is capable of solubilizing the antifungal active agent. The head groups are capable of physically and/or sterically interacting with the antifungal active agent, the alcohol and the polyol, thus allowing formation of the nanodomains. Depending on the antifungal active agent to be loaded into the formulation, the hydrophilic surfactants may include, ionic, cationic zwitterionic or non-ionic surfactants having a hydrophilic nature (*i.e.* having large head groups), thereby providing a surfactant having an affinity for water. Exemplary surfactants are ethoxylated fatty alcohols (cetosteareths, ceteths, oleths, steareths), polysorbates (polysorbate 20, 60, 80), ethoxylated fatty acids (ethoxylated stearate), ethoxylated (hydrogenated/non-hydrogenated) castor oil, ethoxylated glycerides of fatty acids, PEGylated octylphenyl ethers, sugar esters, polyglycerol esters, polyethylene glycols (PEG), copolymers of ethylene oxide (EO) and propylene glycol (PG) moieties, lysolecithins, and combinations thereof.

By some embodiments, the formulation comprises at least two hydrophilic surfactants, *i.e*. at least a first hydrophilic surfactant and at least a second hydrophilic surfactant, the first and second hydrophilic surfactants being different one from the other. The oily phase comprises at least two hydrophilic surfactants. The hydrophilic surfactants are selected and matched such that their combination forms a *"Sherman complex".* The Sherman complex refers to a set of two or more surfactants that form dense, well-packed and compacted interfacial layer, resulting from a match of the two surfactants with two lipophilic tails; namely, one having a longer tail and the other having a shorter tail that are integrated one into the other in the core of the domain. In the Sherman complex, the two surfactants have hydrophilic head groups, the first with a larger head group and the other with a smaller head group, forming strong hydrogen bonding between the head groups. Such complexes provide increased solubilization of the antifungal active agent in the nanodomains and better chemical stabilization of the antifungal active agent within the tails of the surfactants.

According to some embodiments, each of the first and second hydrophilic surfactants is independently selected from polysorbates (ethoxylated sorbitan fatty acid esters), ethoxylated fatty alcohols (*e.g*. Brijs), ethoxylated castor oils, ethoxylated glycerides of fatty acids, ethoxylated fatty acids, and combinations thereof.

According to some embodiments, the formulation comprises at least one first hydrophilic surfactant is a polysorbate or ethoxylated castor oil, and at least one second hydrophilic surfactant is an ethoxylated fatty alcohols, ethoxylated fatty acids, or ethoxylated glycerides of fatty acids.

By some embodiments, the formulation comprises a total amount of hydrophilic surfactants of at least about 8 wt%. According to other embodiments, the formulation comprises between about 8 wt% and about 25 wt% of hydrophilic surfactants.

In some embodiments the ratio between the at least one first hydrophilic surfactant and the at least one second hydrophilic surfactant is between about 1:2 and about 5:1 (w/w).

In some embodiments, the at least one first hydrophilic surfactant is present in the formulation in an amount ranging between about 8 and about 14 wt%, while the at least one second hydrophilic surfactant is present in an amount ranging between about 1 and about 6 wt%.

As noted, in addition to the at least one hydrophilic surfactant, the nanodomains comprise at least one alcohol and at least one polyol. Within the context of the present disclosure, *alcohol* is meant to denote an organic compound that carries a hydroxyl functional group bound to a saturated carbon atom, while *polyol* is meant to denote an organic compound comprising two or more hydroxyl groups.

The combination of at least one alcohol and at least one polyol permits obtaining stabilization of the interface between the nanodomains and the aqueous phase, by cooperating with the hydrophilic surfactant(s) to form a dense and complete interface. The combination of hydrophilic surfactant(s), alcohol(s) and polyol(s) provide suitable solubilization conditions for the progressive migration of the surfactants to the interface upon dilution with the aqueous phase. At low water contents the alcohols and polyols are essential components to render the behavior of the hydrophilic surfactant to be lipophilic-like, adjusting its effective critical packing parameter (ECPP) to >1.3. At higher water levels (e.g. 40-50%), the alcohols and polyol "push" the surfactants to the interface and causing a significant alternation of the ECPP to <0.5. In other words, the alcohols and the polyols control and adjust the hydrophilicity/lipophilicity of the surfactants in accordance with the content of water in the formulation. Thus, the combination of alcohols and polyols allows for complete geometrical packing of the interface, filling the voids in between the surfactants at the interface. Without wishing to be bound by theory, following application on nail/skin and water/volatiles evaporation, the nanodomains rearrange within the polymeric matrix, where the spaces are occupied by the non-volatile components (glycerol, PG, PEG). It is estimated that the polyols function as hydrating agents and/or form part of the continuous phase for the nanodomains during evaporation of the volatile components and formation of the film.

Further, without wishing to be bound by theory, in the structure of the interface of the nanodomain, the alcohol is typically located deeper within the interface, and functions to provide the elasticity of the curvature (R^{e}) and spontaneous curvature (R^{s}, or R^{o}) of the nanodomains' interface with the aqueous phase. The polyol is located closer to the surfactant head groups, thereby dehydrating them and promoting solubilization of the antifungal active agent within the droplets.

According to some embodiments, the at least one alcohol is selected from short-chain aliphatic alcohols and aromatic alcohols. By some embodiments, the at least one alcohol is selected from ethanol, propanol, isopropanol, butanol, benzyl alcohol, transcutol, and combinations thereof.

By some embodiments, the formulation comprises at least two alcohols.

By some embodiments, the formulation comprises a total amount of alcohols of at least about 12 wt%. By some other embodiments, the formulation comprises between about 12 wt% and about 30 wt% of alcohols.

According to some embodiments, the weight ratio between the total amount of hydrophilic surfactants and the total amounts of alcohols in the formulation ranges between about 1:1 and about 1:3.

According to some embodiments, the at least one polyol is selected from propylene glycol, glycerol, xylitol, sorbitol, and other monomeric or dimeric sugar units, polyethylene glycol (PEG), 1,3-propanediol, butylene glycol (1,2-butanediol), pentylene glycol (1,2-penranediol), 1,2-octanediol, and combinations thereof.

By some embodiments, the formulation comprises a total amount of polyols of at least about 2 wt%. By some other embodiments, the formulation comprises between about 2 wt% and about 12 wt% of polyols.

According to some embodiments, the weight ratio between the total amount of hydrophilic surfactants and the total amounts of polyols in the formulation ranges between about 1:1 and about 5:1.

According to some other embodiments, the weight ratio between the total amount of alcohols and the total amount of polyols in the formulation ranges between about 1:1 and about 10:1.

By some embodiments, the nanodomains further comprise at least one solvent, different from said at least one alcohol. The solvent typically functions to increase the solubilization of the antifungal active agent within the nanodomain, thereby enabling increasing the maximal concentration of the antifungal active agent that can be stably loaded into the nanodomains. By some embodiment, the at least one solvent is selected from transcutol, dimethyl sulfoxide (DMSO), ethanol, dimethyl formamide (DMF), tetrahydrofuran (THF), dimethylacetamide (DMA), N-Methyl-2-pyrrolidone (NMP), amines, and combinations thereof. By some other embodiment, the at least one solvent is selected from transcutol, dimethyl sulfoxide (DMSO), ethanol, and combinations thereof.

By some embodiments, the total amount of solvents in the formulation is at most 16 wt%, typically ranging between about 3 wt% and about 10 wt%.

As noted, the aqueous continuous phase of the formulation comprises water, at least one film-forming agent in an amount of no more than about 1 wt% of the formulation, and at least one keratolytic agent.

The *keratolytic agent* is a compound that causes softening and/or at least partial disintegration of the keratinous tissue to facilitate improved permeation of the nanodomains and/or antifungal active agent contained therein into the keratinous tissue. According to some embodiments, the keratolytic agent is selected from glycerol, urea, lactic acid, dimethylsulfoxide (DMSO), dimethicone, alpha-hydroxy acids (salicylic acid *etc*.), or any combination thereof.

By some embodiments, the total amount of keratolytic agents in the formulations is at most 15 wt%, *e.g*. ranging between about 5 wt% and about 14 wt%.

As also noted, the total amount of water in the formulation ranges between about 35 wt% and about 55 wt%. The content of water has a significant impact on the viscosity and structure of the formulation. Higher water content will cause the viscosity of the formulation to significantly decrease, reducing the contact time between the formulation and the keratinous tissue, and hindering the formulation's adherence onto the keratinous tissue. Low water content will prevent spontaneous formation of nanodomains within the aqueous phase, as the high relative amounts of film-forming agent and keratolytic agents in low contents of water will hinder spontaneous arrangement of the hydrophilic surfactants, alcohols and polyols into a stable nanostructure. Further, lower water content will cause a sharp increase in the viscosity of the aqueous phase, damaging the spreadability of the formulation onto the tissue.

The aqueous phase comprises at least one film-forming agent. The *film-forming agent* is a compound that causes an increase in the viscosity of the aqueous phase to a desired viscosity, while functioning to form a thin film when applied onto the keratinous tissue once volatile components are evaporated from the formulation after application. The film-forming agent typically forms a 3-dimensional network of macromolecules, for example a viscoelastic network of polymeric chains, in which the nanodomains are embedded and homogenously dispersed, thereby increasing the viscosity and modifying the rheological behavior of the aqueous phase.

The formulations of this disclosure are formulated to form a film onto the keratinous tissue once applied thereon, with the nanodomains embedded within the film. In other words, the formulation comprises at least 10 wt% of volatile components, that can evaporate once the formulation is applied onto the keratinous tissue, permitting the remainder components of the formulation residing on the tissue to form a film, that adheres to the keratinous tissue of a prolonged period of time, permitting release of the nanodomains and the antifungal active agent therefrom to the keratinous tissue. The term *volatile component* means to denote a compound, different from water, that is capable of substantive evaporation at atmospheric pressure and at a temperature of at least about 30°C.

By some embodiments, the at least one film-forming agent is selected from water-soluble or colloidal water-soluble polymers (hydro-colloids), such as cellulose ethers (*e.g.* hydroxyethyl cellulose, methyl cellulose, hydroxypropylmethyl cellulose), polyvinylalcohol, polyquaternium-10, guar gum, hydroxypropyl guar gum, xanthan gum (such as Keltrals, Xanturals such as Xantural 11K, Xantural 180K, Xantural 75 and others), gellans (Kelogels), gelatin and gelatin derivatives, Aloe vera gel, amla, carrageenan, oat flour, starch and modified starch (from corn rice or other plants), gelatin (from porcine or fish skin), ghatty gum, gum Arabic, inulin (from chicory), Konjac gum, locust bean gum (LBG), fenugreek, marshmallow root, pectin (high and low methoxy) and modified pectins, quinoa extract, red alga, solagum, tragacanth gum (TG) and any mixtures thereof.

By some other embodiments, the film-forming agent is selected amongst acrylic acid/ethyl acrylate copolymers and the carboxyvinyl polymers under the trademark of Carbopol resins. Examples include Carbopol 934, Carbopol 940, Carbopol 950, Carbopol 980, Carbopol 951 and Carbopol 981. Carbopol 934 is a water-soluble polymer of acrylic acid crosslinked with polyallyl ether of sucrose having an average of about 5.8 allyl groups for each sucrose molecule. Also suitable for use are hydrophobically-modified crosslinked polymers of acrylic acid having amphipathic properties available under the Trade Name Carbopol 1382, Carbopol 1342 and Pemulen TR-1. A combination of the polyalkenyl polyether cross-linked acrylic acid polymer and the hydrophobically modified crosslinked acrylic acid polymer may also be suitable.

According to some embodiments, the film-forming agent is selected from xanthan gum, gellan, sodium alginate, pectin, low and high methoxy pectins, carbomers, and mixtures thereof.

According to other embodiments, the film-forming agent is xanthan gum or gellan.

According to some embodiments, the formulation comprises up to about 0.5 wt% of the at least one film-forming agent. By some embodiments, the formulation comprises between about 0.05 wt% and about 0.5 wt% of the at least one film-forming agent.

In some embodiments, the film obtained onto the keratinous tissue after application of the formulation is substantially transparent.

By some other embodiments, the film obtained onto the keratinous tissue after application of the formulation comprises no more than about 2 wt% film-forming agent.

According to another aspect of this disclosure, there is provided a formulation for forming a depot of an antifungal active agent over a keratinous tissue, the formulation comprising nanodomains dispersed in an aqueous continuous phase. The nanodomains comprise said antifungal active agent, at least one hydrophilic surfactant, at least one alcohol, and at least one polyol. The aqueous phase comprises water, at least one film-forming agent in an amount of no more than about 1 wt%, and at least one keratolytic agent, the total amount of water in the formulation being between about 35 wt% and about 55 wt%. The formulation is formulated to form a film with the nanodomains embedded therein, once applied onto said keratinous tissue, the film having a residence time on the keratinous tissue of between about 1 hour and 24 hours.

In other words, the formulation is designed to form a film, having properties to permit residence time onto the keratinous tissue, once applied thereon, as to form a reservoir of the nanodomains onto the tissue, to permit increased contact time and increase in the amount of antifungal active agent delivered to the keratinous tissue during contact.

By another aspect, there is provided a formulation for topical delivery of an antifungal active agent to a keratinous tissue, the formulation comprising nanodomains dispersed in an aqueous continuous phase, the nanodomains comprising said antifungal active agent, at least one hydrophilic surfactant, at least co-surfactant, and optionally at least one solvent, the aqueous phase comprising water, at least one film-forming agent in an amount of no more than about 1 wt%, at least one keratolytic agent and at least one solvent, the total amount of water in the formulation being between about 35 wt% and about 55 wt%.

As noted, the formulation is designed for the spontaneous formation of the nanodomains once the components of the nanodomains are introduced into the aqueous phase. Hence, the components of the formulation may be maintained separately, and the formulation can be prepared shortly before use. Thus, by another aspect, this disclosure provides a kit for preparing a formulation for delivery of an antifungal active agent to a keratinous tissue, the kit comprising: a first container holding a concentrate that comprises said antifungal active agent, at least one hydrophilic surfactant, at least one alcohol, and at least one polyol; a second container holding an aqueous solution comprising water, at least one film-forming agent in an amount of no more than about 1 wt% of the total formulation, and at least one keratolytic agent; and means of mixing the concentrate with the aqueous solution to obtain a topical formulation in the form of nanodomains dispersed in an aqueous continuous phase; the kit being configured to dose the concentrate and the aqueous solution such that the total amount of water in the formulation is between about 35 wt% and about 55 wt%.

In other words, the concentrate (comprising the antifungal active agent, at least one hydrophilic surfactant, at least one alcohol, and at least one polyol) can be maintained separately from the aqueous solution, and before application - the kit is configured to permit mixing of the concentrate with the aqueous solution in order to obtain, *in situ,* the formulation with the structure of nanodomains dispersed in an aqueous continuous phase.

The mixing means can be active, *e.g*. mechanically or electrically operated mixer. Alternatively, the mixing can be obtained by one or more static mixers - *i.e.* a mechanical structure that permits simultaneous introduction of the concentrate and the aqueous solution thereinto, and under forced flow through a static mixing arrangement (*e.g*. non-moving baffles or flow diverters), causes intimate intermixing of the concentrate and the aqueous solution without operation of high shear forces to obtain the formulation.

By controlling the doses of concentrate and aqueous solution that are introduced into the mixing means, the final content of water in the formulation to be dispensed can be controlled.

For example, the kit can comprise a housing, holding therein the first and second containers, and configured with a mixing arrangement located in the housing adjacent an application means. The housing can further comprise one or more dosing means, permitting controlled dosing of the concentrate and the aqueous solution into the mixing arrangement to ensure proper final content of water in the formulation.

By another aspect, there is provided an applicator for topically applying a formulation as described herein to a keratinous tissue, the applicator comprising at least one compartment holding said formulation and means of application of the formulation onto the keratinous tissue.

By yet another aspect, there is provided an applicator for topically applying a formulation as described herein to a keratinous tissue, the applicator comprising: a first container holding a concentrate that comprises said antifungal active agent, at least one hydrophilic surfactant, at least one alcohol, and at least one polyol; a second container holding an aqueous solution comprising water, at least one film-forming agent in an amount of no more than about 1 wt% of the total formulation, and at least one keratolytic agent; means of mixing the concentrate with the aqueous solution to obtain said formulation in the form of nanodomains dispersed in an aqueous continuous phase; and means of application of the formulation onto the keratinous tissue; the applicator being configured to dose the concentrate and the aqueous solution such that the total amount of water in the formulation is between about 35 wt% and about 55 wt%.

A further aspect of this disclosure provides a method (not claimed) of delivering an antifungal active agent to a keratinous tissue, the method comprising topically applying a formulation as described herein on said keratinous tissue.

By a further aspect, there is provided a method of treating a fungal infection (not claimed) in a keratinous tissue, the method comprising topically applying a formulation as described herein on said keratinous tissue.

*Treatment* or any lingual variation thereof, as used herein, refers to the administering of a therapeutic amount of the formulation described herein, which is effective to ameliorate undesired symptoms associated with a fungal infection, to prevent the manifestation of such symptoms before they occur, to slow down the progression of the fungal infection, slow down the deterioration of symptoms, to enhance the onset of remission period, slow down the irreversible damage caused in the progressive stage of the fungal infection, to delay the onset of said progressive stage, to lessen the severity or cure the fungal infection, or to prevent the infection from reoccurring or a combination of two or more of the above.

As known, the *effective amount* for purposes herein may be determined by such considerations as known in the art. The effective amount is typically determined in appropriately designed clinical trials (dose range studies) and the person versed in the art will know how to properly conduct such trials in order to determine the effective amount. As generally known, the effective amount depends on a variety of factors including the distribution profile within the body, a variety of pharmacological parameters, undesired side effects, if any, factors such as age and gender, and others.

According to some embodiments, the formulations can be used for treating conditions associated with dermatophytes and non-dermatophytes, including *Tinea unguium, Trichophyton rubrum, Trichophton mentagrophytes, Trichophyton interdigitale, Trichophyton tonsurans, Trichophyton violaceum, Trichophyton schoenleinii, Trichophyton megninii, Trichophyton soudanense, Trichophyton yaoundei, Microsporum audouinii, Microsporum ferrugineum, Nannizzia gypsea, Epidermophyton floccosum, Candida albicans, Fusarium species, Aspergillus, Acremonium* species, *Scytalidium* species, *Scopulariopsis brevicaulis,. Microsporum canis, Trichophyton equinum, Trichophyton erinaceid, Trichophyton verrucosum, Microsporum nanum, Microsporum distortum, Microsporum gypseum, Sporothrix schenckii, Malassezia furfur* and *Onychocola canadensis.*

According to another aspect, there is provided a method of treating *Tinea unguium* infection (not claimed) in a keratinous tissue, the method comprising topically applying a formulation as described herein on said keratinous tissue.

By another aspect, there is provided a formulation as described herein for use in treating a fungal infection in a keratinous tissue.

By yet another aspect, there is provided a formulation as described herein for use in treating *Tinea unguium* infection in a keratinous tissue.

The phrases *ranging*/*ranges between* a first indicate number and a second indicate number and "ranging/ranges from" a first indicate number "to" a second indicate number are used herein interchangeably and are meant to include the first and second indicated numbers and all the fractional and integral numerals there between. It should be noted that where various embodiments are described by using a given range, the range is given as such merely for convenience and brevity and should not be construed as an inflexible limitation on the scope of the invention. Accordingly, the description of a range should be considered to have specifically disclosed all the possible sub-ranges as well as individual numerical values within that range.

As used herein, the term *about* is meant to encompass deviation of ±10% from the specifically mentioned value of a parameter, such as temperature, pressure, concentration, *etc.*

Unless the context requires otherwise, the term *comprise,* and variations such as *comprises* and *comprising,* is to be understood to imply the inclusion of a stated integer or step or group of integers or steps but not the exclusion of any integer or step or group of integers and steps.

Generally, it is noted that the phrase ... *at least one ...* as applied to any component of a formulation disclosed herein should be read to encompass one, two, three, four, or even more different occurrences of said component in the formulation.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** are pictures of exemplary compositions of this disclosure.
**Figs. 2A-2I** are transmission profiles obtained by LUMiSizer^{®} for several formulations: AP950 (Fig. 2A), AP_05 (Fig. 2B), MUR_5A (Fig. 2C), MUR_5B (Fig. 2D), MUR_5C (Fig. 2E), AP4500 (Fig. 2F), AP4500LX (Fig. 2G), NK003 (Fig. 2H), and REAP4500 (Fig. 2I).
**Figs. 3A-3I** show the droplet size distribution obtained by DLS measurements for several formulations: AP950 (Fig. 3A), AP_05 (Fig. 3B), MUR_5A (Fig. 3C), MUR_5B (Fig. 3D), MUR_5C (Fig. 3E), AP4500 (Fig. 3F), AP4500LX (Fig. 3G), NK003 (Fig. 3H), and REAP4500 (Fig. 3I).
**Figs. 4A-4D** show cryo-TEM images for the following formulations: MUR_05 (Fig. 4A), MUR_5C (Fig. 4B), AP4500 (Fig. 4C), and AP4500LX (Fig. 4D). Arrows indicate free-water crystallization.
**Figs. 5A-5G** show the viscosity as a function of shear rate of some of the formulations: AP950 (Fig. 3A), AP_05 (Fig. 3B), MUR_5A (Fig. 3C), MUR_5B (Fig. 3D), MUR_5C (Fig. 3E), AP4500 (Fig. 3F), and AP4500LX (Fig. 3G).
**Figs. 6A-6F** provide visual appearance of empty and TRB-loaded AP950 formulation upon storage of up to 9 months at 25°C and up to 6 months at 40°C: 2.4% TRB-loaded at 25°C (Fig. 6A); 2.4% TRB-loaded at 40°C (Fig. 6B); empty vehicle at 25°C (Fig. 6C); empty vehicle at 40°C (Fig. 6D); 2.4% TRB-loaded at 25°C in pen applicator (Fig. 6E); and 2.4% TRB-loaded at 40°C in pen applicator (Fig. 6F).
**Fig. 7** is a picture of a nail prepared for penetration test: Position "a" - where the sample was in contact with the nail, and Position "b" - an adjacent position where the sample was not in contact with the nail.
**Figs. 8A-8E** show average overall nail involvement (Fig. 8A) and percentage of subjects with overall nail involvement at time 0, 6 weeks, 12 weeks and 16-24 weeks (Figs. 8B-8E, respectively), during treatments of subjects treated with a 2.4% terbinafine hydrochloride Test Formulation according to this disclosure, during Phase I/IIa clinical studies.
**Fig. 9** shows average nail thickness measured during Phase I/IIa clinical studies of subjects treated with the Test Formulation.
**Fig. 10** shows average of overall nail involvement (in %) as a function of time during Phase I/IIa clinical studies of subjects treated with the Test Formulation.
**Fig. 11** shows complete cure rates for the Test Formulation compared to several commercial products.

### DETAILED DESCRIPTION OF EMBODIMENTS

### Exemplary compositions

Shown in **Tables 1-1** and **1-3,** are exemplary formulations according to this disclosure. The formulations were prepared according to the following protocol: a concentrate solution is prepared by mixing, at room temperature or slightly elevated temperature (*e.g*. 40-60°C) the surfactants, co-surfactants / solvents and penetration enhancer, to obtain a homogenous solution. Then, terbinafine hydrochloride (TRB) was added. TRB was chosen as a model molecule to show the capacity of the compositions to deliver antifungal agents to the nail area.

The aqueous phase was prepared separately by first dissolving the film-forming polymer and then adding the keratolytic agents. Finally, the pH was adjusted to *ca*. 3.8-4.2 by adding one or more organic bases. The concentrate and the aqueous phase were then mixed at room temperature to obtain the final nanostructure.

**Table 1-1: Exemplary formulations**

| **Component** | | **AP950** | **AP950C** | **AP3000** | **AP3500** | **AP4500** | **AP4500 LX** |
|---|---|---|---|---|---|---|---|
| Brij cs 20 | surfactant | 2.76 | 2.76 | 2.76 | 2.76 | 2.76 | 2.76 |
| Tween 20 | | 5.52 | 5.52 | 5.52 | 5.52 | 5.52 | 5.52 |
| Tween 60 | | 4.69 | 4.69 | 4.69 | 4.69 | 4.69 | 4.69 |
| Benzyl alcohol | Co-surfactant / co-solvent / solvent | 1.66 | 1.66 | 0.66 | 1.66 | 1.66 | 1.66 |
| Transcutol | | 3.86 | 3.86 | 3.86 | 3.86 | 3.86 | 3.86 |
| EtOH | | 12.76 | 12.76 | 12.76 | 7.76 | 12.76 | 12.76 |
| IPA | | 3.59 | 3.59 | 3.59 | 3.59 | 5.59 | 5.59 |
| Propylene Glycol | | 2.76 | 2.76 | 2.76 | 3.76 | 3.76 | 3.76 |
| PEG400 | | --- | --- | 2 | --- | 4 | 4 |
| DMSO | Penetrating enhancer | 5 | 10 | 1 | 1 | --- | --- |
| Terbinafine | API | 2.4 | 2.4 | 2.4 | 2.4 | 2.4 | 2.4 |
| Water | | 45.8 | 45.8 | 46.8 | 50.8 | 40.8 | 40.85 |
| Xanthan gum | Film-forming agent | 0.2 | 0.2 | 0.2 | 0.2 | 0.2 | 0.15 |
| Glycerol | Keratolytic agent | 5 | --- | 5 | 5 | 6 | 6 |
| Urea | | 1 | 1 | 3 | 2 | 3 | 3 |
| Lactic acid | | 3 | 3 | 3 | 3 | 3 | 3 |
| Dimethicone | | --- | --- | --- | 2 | --- | --- |
| **Total** | | **100** | **100** | **100** | **100** | **100** | **100** |
| **Total concentrate** | | **45** | **50** | **42** | **32** | **47** | **47** |
| **Total aqueous phase** | | **55** | **50** | **58** | **68** | **53** | **53** |

**Table 1-2: Exemplary formulations**

| **Component** | | **AP_05** | **MUR-05A** | **MUR-05B** | **MUR-05C** | **MUR-05D** |
|---|---|---|---|---|---|---|
| Brij cs 20 | surfactant | 3.68 | 3.68 | 3.68 | 3.68 | 3.68 |
| Tween 20 | | 6 | 6 | 6 | 6 | 6 |
| Tween 60 | | 5.69 | 5.69 | 5.69 | 5.69 | 5.69 |
| Benzyl alcohol | Co-surfactant / co-solvent / solvent | 1.66 | 1.66 | 1.66 | 1.66 | 1.66 |
| Transcutol | | 3.86 | 3.86 | 3.86 | 3.86 | 3.86 |
| EtOH | | 12.76 | 13.76 | 13.76 | 13.76 | 12.76 |
| IPA | | 3.59 | 3.59 | 3.59 | 3.59 | 3.59 |
| Propylene Glycol | | 2.76 | 2.76 | 2.76 | 3.76 | 2.76 |
| PEG400 | | --- | --- | 3 | 4 | --- |
| Terbinafine | API | 5 | 5 | 5 | 5 | 5 |
| Water | | 45.8 | 45.8 | 45.8 | 40.8 | 45.8 |
| Xanthan gum | Film-forming agent | 0.2 | 0.2 | 0.2 | 0.2 | 0.2 |
| Glycerol | Keratolytic agent | 5 | 5 | 5 | 5 | 5 |
| Urea | | 1 | 1 | 0 | 1 | 1 |
| Lactic acid | | 3 | 2 | 0 | 2 | 3 |
| **Total** | | **100** | **100** | **100** | **100** | **100** |
| **Total concentrate** | | **35** | **35** | **38** | **40** | **35** |
| **Total aqueous phase** | | **65** | **65** | **62** | **60** | **65** |

**Table 1-3: Exemplary formulations**

| **Component** | | **NK0003** | **REAP 4500** | **REAP 4500IPA** | **NK003 NU** | **NK003 NL** | **NL003 NT** |
|---|---|---|---|---|---|---|---|
| Brij cs 20 | surfactant | 2.76 | - | - | 2.76 | 2.76 | 2.76 |
| Tween 20 | | 5.51 | 5.95 | 5.95 | 5.51 | 5.51 | 5.51 |
| Tween 60 | | 4.68 | 5.16 | 5.16 | 4.68 | 4.68 | 4.68 |
| Benzyl alcohol | Co-surfactant / co-solvent / solvent | 1.66 | 2.20 | 2.20 | 1.66 | 1.66 | 1.66 |
| Transcutol | | 3.85 | 4.29 | 4.29 | 3.85 | 3.85 | 3.85 |
| EtOH | | 12.74 | 18.32 | - | 12.74 | 12.74 | 12.74 |
| IPA | | 5.58 | - | 18.32 | 5.58 | 5.58 | 5.58 |
| Propylene Glycol | | 3.75 | 4.19 | 4.19 | 3.75 | 3.75 | 3.75 |
| PEG400 | | 3.99 | 4.43 | 4.43 | 3.99 | 3.99 | 3.99 |
| Terbinafine | API | 2.47 | 2.47 | 2.47 | 2.47 | 2.47 | 2.47 |
| Water | | 47.50 | 38.99 | 38.99 | 41.94 | 41.94 | 40.74 |
| Xanthan gum | Film-forming agent | 0.21 | 0.15 | 0.15 | 0.15 | 0.15 | 0.15 |
| Glycerol | | 5.19 | 6 | 6 | 6 | 6 | |
| Urea | Keratolytic agent | - | 3 | 3 | - | 3 | 3 |
| Lactic acid | | - | 3 | 3 | 3 | - | 3 |
| Butylated hvdroxvtoluene | Additives | 0.06 | 0.06 | 0.06 | 0.06 | 0.06 | 0.06 |
| EDTA | | 0.05 | 0 | 0 | 0.05 | 0.05 | 0.05 |
| Triethanol amine | | 0 | 1.80 | 1.80 | 1.80 | 1.80 | 0 |
| **Total** | | **100** | **100** | **100** | **100** | **100** | **100** |
| **Total concentrate** | | **47** | **47** | **47** | **47** | **47** | **47** |
| **Total aqueous phase** | | **53** | **53** | **53** | **53** | **53** | **53** |

The compositions were characterized to assess their physical properties, chemical stability under various conditions, as well as performance, as will now be detailed.

### Physical characterization

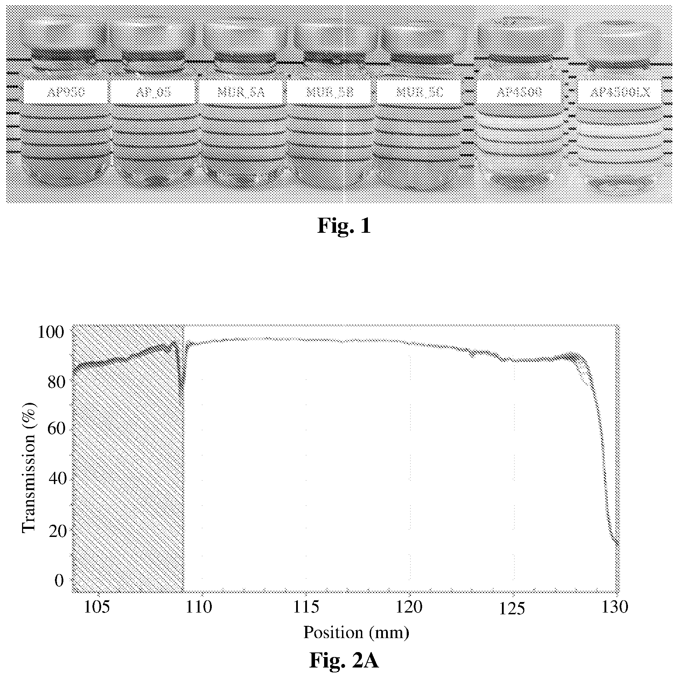

All tested formulations showed a nanostructure of surfactants-based nanodomains, homogenously dispersed in the aqueous phase. The formulations exhibited high transparency and homogeneity, as seen in **Fig. 1****,** as well as thermodynamic stability.

### LUMiSizer^{®} tests

The physical stability of the formulations was assessed by LUMiSizer^{®}, an analytical centrifuge that monitors the light transmission through the samples while the sample are being centrifuged horizontally. The changes in transmission indicate the stability of the sample - when the transmission profile remains constant, the sample is considered physically stable, and its shelf-life can be extrapolated based on the measurement conditions. For measurement conditions employed, the extrapolated shelf life is at least 2 years.

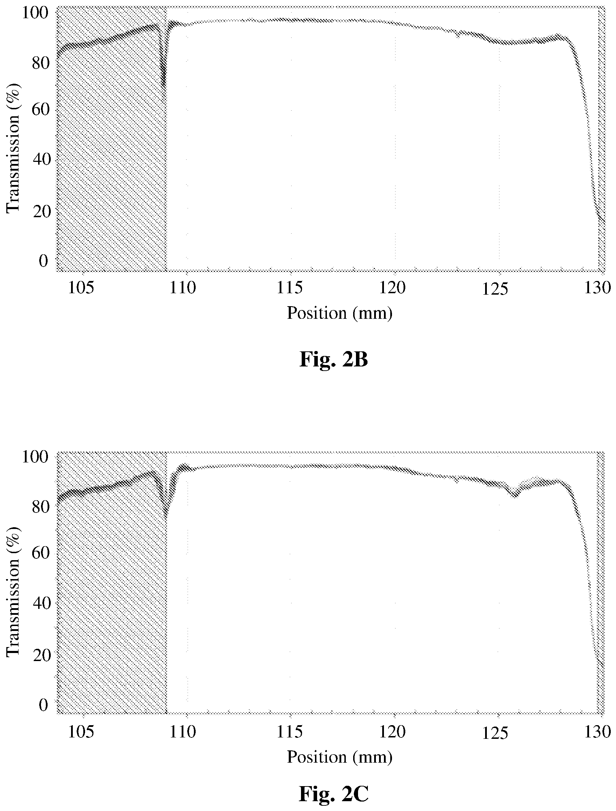

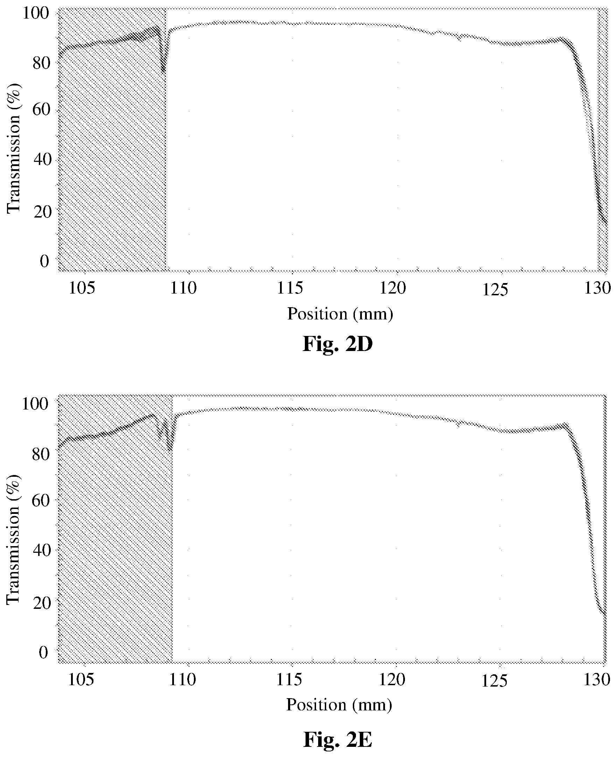

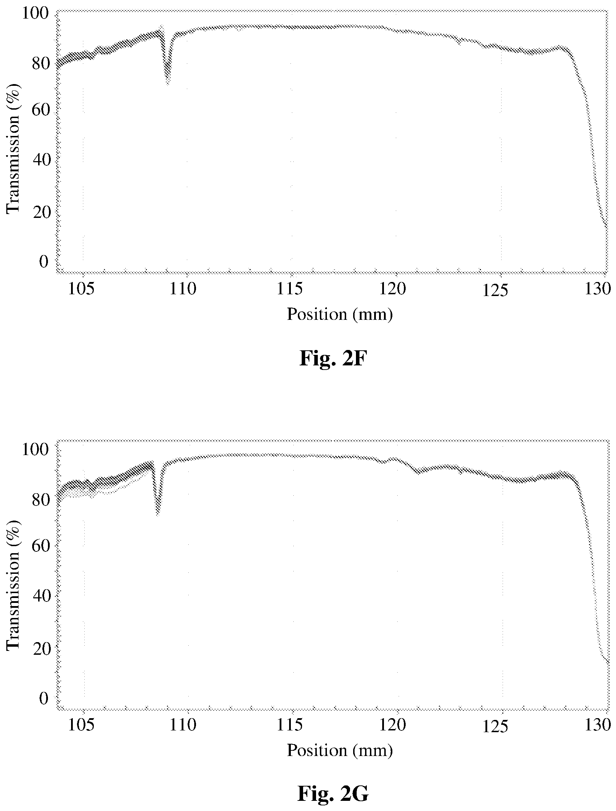

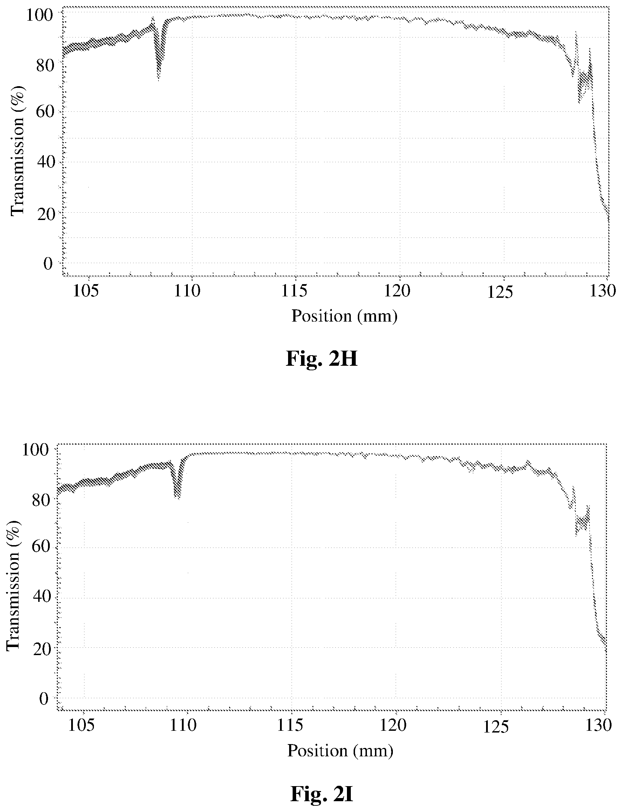

The transmission profiles of some of the formulations, showing the transmission percent as a function of measurement position and time are shown in **Figs. 2A-2I**. As can clearly be seen, in all tested formulations no changes in transmission were observed (*i.e.* full transmission was observed), indicating that all formulations are stable and are expected to maintain stability for at least 2 years from preparation.

### Droplet size

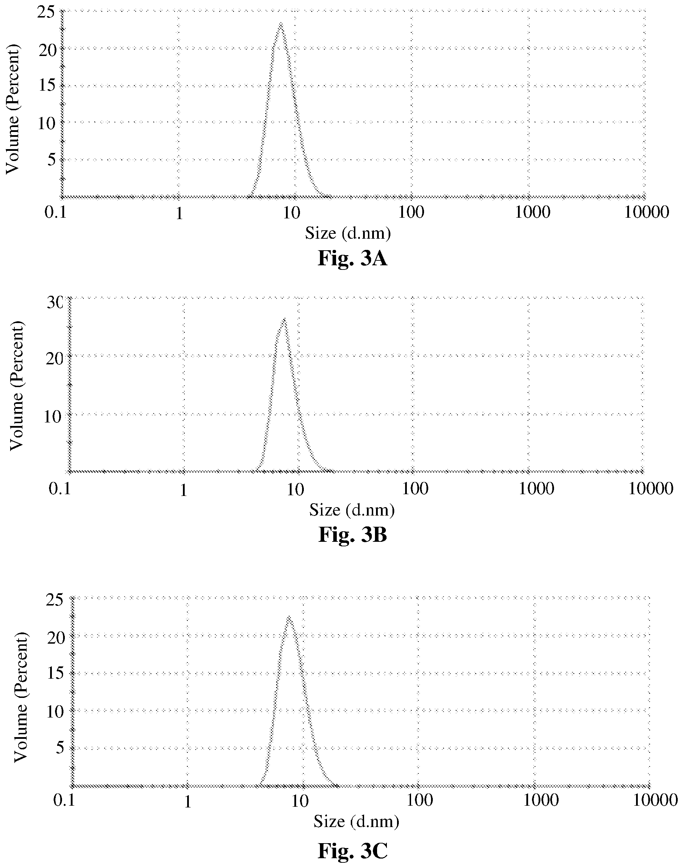

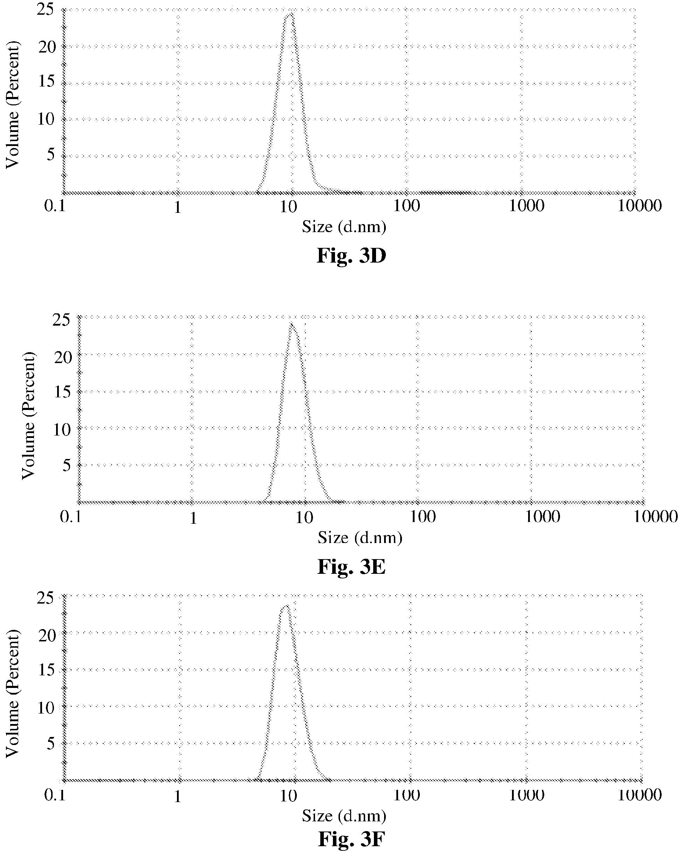

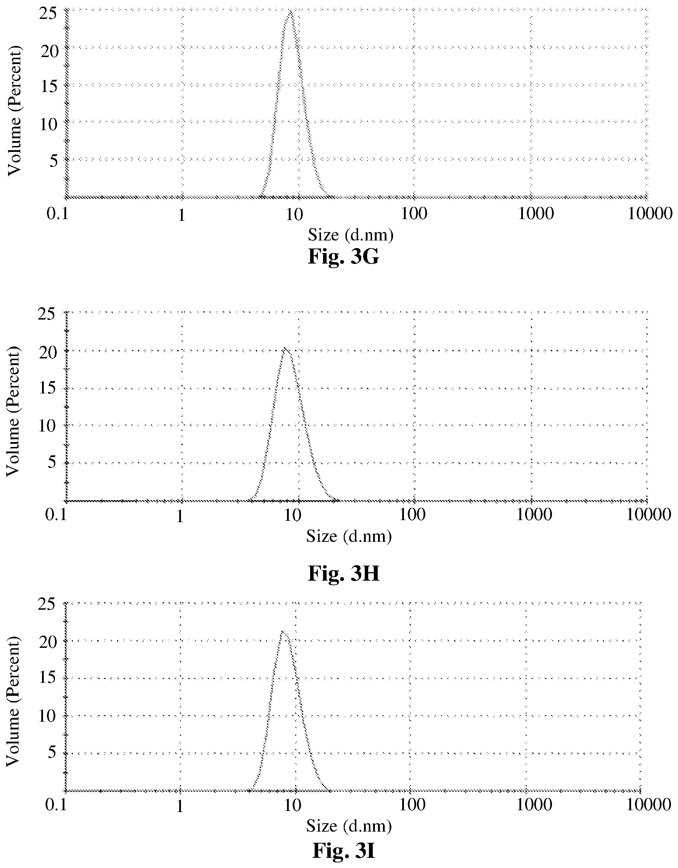

To confirm the presence of the nanostructures, the systems were further characterized by Dynamic Light Scattering (DLS) after water dilution, and cryo-TEM (samples were measured without further treatment). **Figs. 3A-3I** show the droplet-size distribution (measured as the volumetric fraction of the droplets). Summary of the DLS analysis results is provided in **Table 2.**

**Table 2: DLS test results**

| **Formulation** | **TRB content (wt%)** | **Droplet size** (nm) | **Population (%vol)** |
|---|---|---|---|
| AP950 | 2.4 | 8.109 | 99.8 |
| AP_05 | 5.0 | 7.986 | 99.7 |
| MUR_5A | 5.0 | 8.396 | 99.9 |
| MUR_5B | 5.0 | 9.896 | 98.5 |
| MUR_5C | 5.0 | 8.518 | 99.5 |
| AP4500 | 2.4 | 8.868 | 99.6 |
| AP4500LX | 2.4 | 8.918 | 99.8 |
| NK003 | 2.47 | 8.676 | 99.6 |
| REAP4500 | 2.47 | 8.673 | 99.9 |

As seen from Table 2 and Figs. 3A-3I, all tested compositions are composed of nano-droplets of *ca.* 8-10nm which count for over 98% of all droplets.

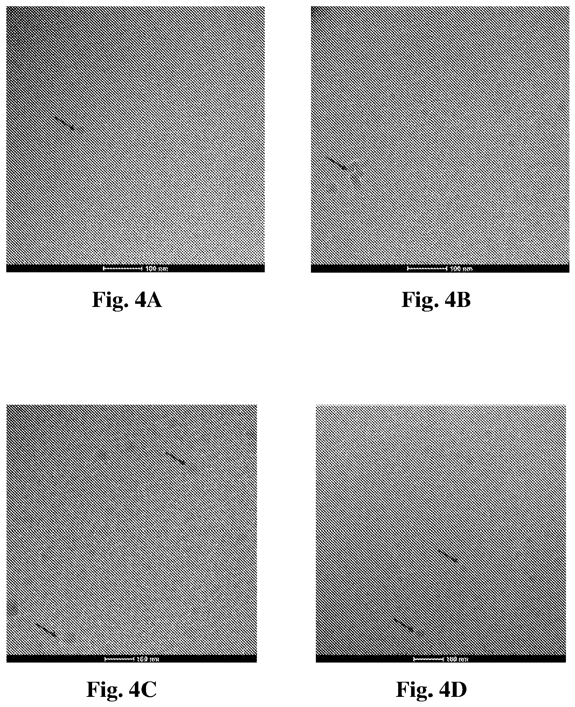

**Figs. 4A-4D** show cryo-TEM images, confirming the presence of the nanodomains. As indicated by the images, formulations of both 2.4% and 5.0% TRB consist of nanostructures of about less than 10nm. Due to the high density of the structures and the limited contrast between the structures and the water in the continuous phase, the shape and dimensions of the nanostructures can only be roughly assessed. The arrows in the images indicate free-water crystallization which typically appears and is related to samples' preparation rather than the actual structure.

### Viscosity

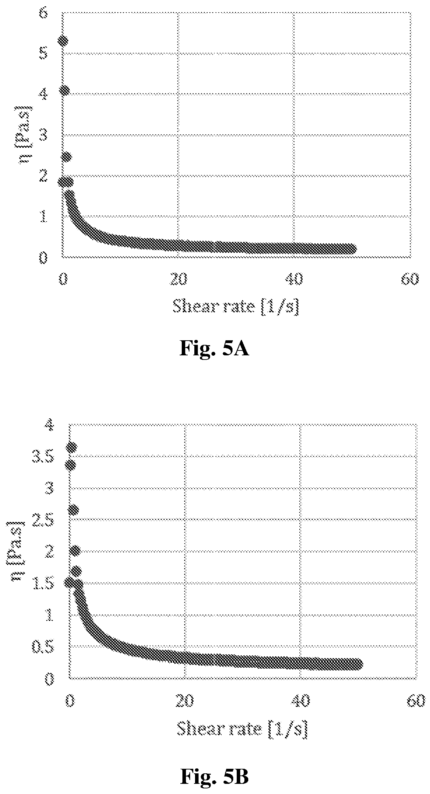

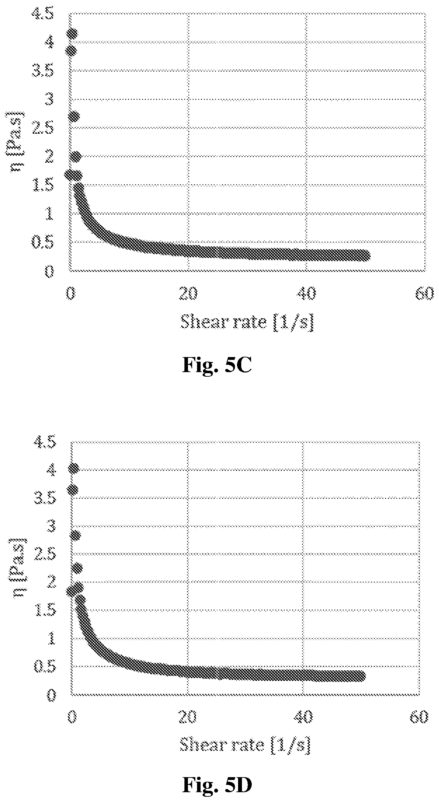

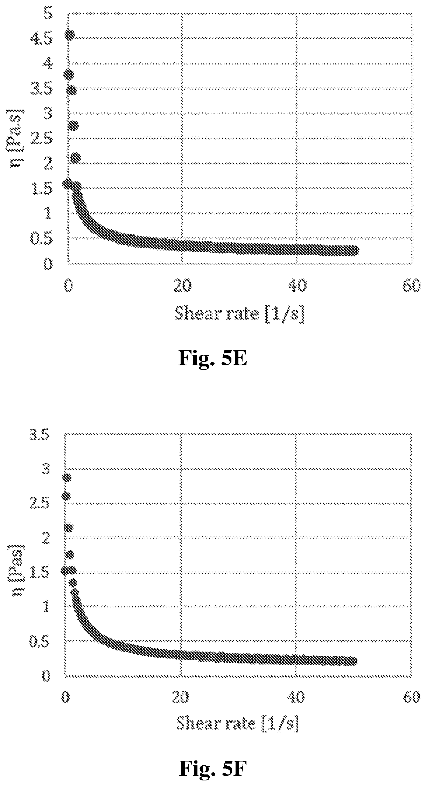

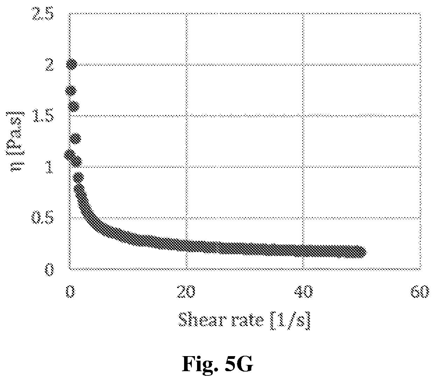

As noted, the formulations of this disclosure need to exhibit sufficiently low viscosity in order to be applied easily, however also be sufficiently viscous to ensure adhesiveness to allow appropriate contact with the nail plate. **Figs. 5A-5G** show the viscosity as a function of shear rate of some of the formulations; **Table 3** show the apparent viscosity values.

The viscosity measurements were conducted using RS6000 rheometer (Thermo Scientific) equipped with C60/1 TiL-L12007 cone. The instrument was operated under rotational mode within shear rates range of 0.00010- 50.00 1/s for 6 minutes. All measurements were conducted at 25±1°C. Under these conditions the formulations possess non-Newtonian behavior, thus, the viscosities were reported for the minimal observed shear rate.

**Table 3: Viscosity test results**

| **Formulation** | **TRB content (wt%)** | **Shear rate (1/s)** | **Apparent viscosity (Pa**•**s)** |
|---|---|---|---|
| AP950 | 2.4 | 0.324 | 4.088 |
| AP_05 | 5.0 | 0.326 | 3.645 |
| MUR_5A | 5.0 | 0.317 | 4.148 |
| MUR_5B | 5.0 | 0.324 | 4.037 |
| MUR_5C | 5.0 | 0.306 | 4.566 |
| AP4500 | 2.4 | 0.339 | 2.867 |
| AP4500LX | 2.4 | 0.319 | 1.538 |

The viscosity profiles demonstrate typical behavior of pseudoplastic, non-Newtonian liquids for which the viscosity decreases as the shear rate increases. Overall, the measured viscosities at low shears varied within the range of *ca.* 3.0-4.5 Pa·s, with the exception of AP4500LX, for which the viscosity was slightly lower due to the lower concentration of xanthan gum (0.15% versus 0.2% in all other compositions).

### Structural analysis by PGSE-NMR

The mobility of the formulations' components was also measured by PGSE-NMR. The diffusion coefficients (×10⁻¹¹) of some of the formulations' components are presented in **Table 4.** The measurements were carried out under gradient/pulses program of δ=15.0ms, Δ=200.0, gmax=40.40. The diffusion coefficients were calculated from a fit function plotted individually for each signal using Dynamic Center software. The reported diffusion coefficients are the mean values calculated for the corresponding peaks of each component.

The surfactants diffusion coefficient is provided as an average for all three surfactants used (Brij CS20, **T**ween 20 and **T**ween 60), while the co-surfactants diffusion coefficient is an average calculated for propylene glycol, ethanol, isopropanol, transcutol, glycerol and PEG 400.

**Table 4: Diffusion coefficients for some of the formulations' components**

| **Sample** | **Diffusion coefficient × 10⁻¹¹ (m²/s)** | | | | | | | |
|---|---|---|---|---|---|---|---|---|
| | **Surfactants** | **Co-surfactants** | **Benzyl alcohol** | **Urea** | **Lactic acid** | **DMSO** | **TRB** | **water** |
| AP950 | 0.89 | 20.62 | 17.67 | 62.90 | 14.38 | 27.50 | 5.71 | 68.80 |
| AP_05 | 0.80 | 19.50 | 15.75 | 56.90 | 22.28 | - | 4.12 | 55.30 |
| MUR_5A | 1.04 | 21.88 | 16.80 | 58.90 | 12.63 | - | 4.63 | 56.50 |
| MUR_5B | 0.61 | 20.26 | 17.72 | - | - | - | 4.31 | 53.70 |
| MUR_5C | 0.67 | 17.46 | 15.32 | 52.70 | 10.82 | - | 4.21 | 47.80 |

As seen from Table 4, by the PGSE-NMR measurements the diffusivity was determined including the surfactants, co-surfactants, benzyl alcohol, urea, lactic acid, DMSO, TRB and water. Overall, no significant differences in the diffusivity of all components were observed between all tested samples, with one exception of MUR_05C which consistently showed lower D values (meaning lower diffusivities) due to the lower content of water (40.8% in MUR_05C compared to 45.8% in all other systems). In all samples, surfactants showed the lowest diffusivity with 0.6-1.0×10⁻¹¹ m²/s.

For all other components beside TRB, the diffusion coefficients were of 10⁻¹⁰ m²/s in magnitude, where urea and water showed the fastest mobility. TRB molecules showed slightly higher diffusivity than that of the surfactants (4.1-5.7×10⁻¹¹ m²/s) suggesting that the TRB is likely to be located at the outer layers of the interface between the surfactants' molecules and the aqueous phase. It was also noticeable that for the formulations loaded with 5% TRB, the measured diffusivity was lower compared to AP950 loaded with 2.4% TRB: 4.1-4.6×10⁻¹¹ m²/s compared to 5.7×10⁻¹¹ m²/s indicating that at higher levels TRB tends to bound to the surfactants.

Lactic acid molecules seem to diffuse slightly slower compared to the urea and co-surfactants. This might indicate that the lactic acid molecules partially interact with other components, possibly with TRB, contributing to its ionization. The absence of urea and lactic acid in composition MUR_05B seems to have no significant effect on the diffusivity of all other components.

Excipients such as lactic acid and urea, which function as keratolytic agents, have a considerable contribution to the potential efficiency of the formulations to treat onychomycosis. As the NMR measurements indicate, the diffusivity of both components was relatively higher, suggesting that both are located (completely or partially) in the continuous phase. Hence, both are accessible to interact with the nail plate and enhance the permeation of the TRB into the nail.

Benzyl alcohol molecules showed slightly lower diffusivity compared to the other co-surfactants, probably due to the hydrophobic nature of the molecules (15.3-17.7×10⁻¹¹ m²/s). Accordingly, it is to anticipate that the molecules solvate the interface and perhaps facilitate the stabilization of the interface in the presence of TRB.

### Chemical stability

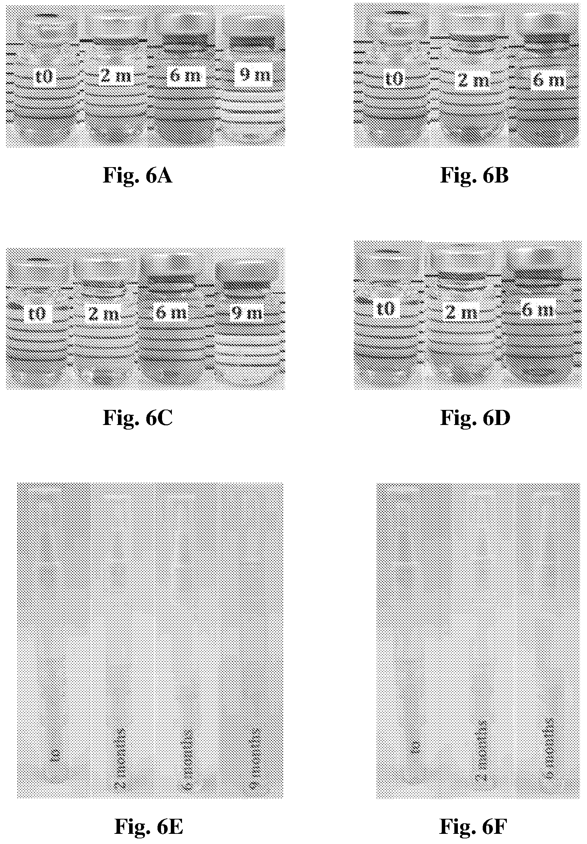

A selected formulation, AP950, was tested for the chemical stability of the TRB at standard (25°C) and accelerated (40°C) conditions. The vehicle (AP950 without TRB) was tested as well as a control sample. As a part of the stability study, the appearance, pH, TRB assay and impurities formation were assessed. For the study, the TRB-loaded compositions were packed in two different packages: glass vial and a pen-applicator. **Figs. 6A-6F** provide visual appearance of empty and TRB-loaded AP950 formulation upon storage of up to 9 months at 25°C and up to 6 months at 40°C.

As seen in Figs. 6A-6F, AP950 (loaded with TRB) showed very slight coloration after 9 months storage at 25°C. The samples that were placed in the pen-applicators showed no coloration after storage at both 25°C and 40°C.

All samples were tested for pH as well. **Tables 5-1** and **5-2** summarize pH values obtained for both loaded and empty AP950 a 25°C and 40°C.

**Table 5-1: pH values for empty and TRB-loaded AP950 stored at 25°C**

| **Sample** | **Package** | **TRB (wt%)** | **pH value** | | | | |
|---|---|---|---|---|---|---|---|
| | | | **t₀** | **t_{2M}** | **t_{6M}** | **t_{9M}** | **Δt₉₋₀** |
| AP950 | Glass | 2.4 | 4.60 | 4.60 | 4.51 | 4.42 | -0.18 |
| AP950 | Pen appl. | 2.4 | 4.60 | 4.54 | 4.51 | 4.44 | -0.16 |
| AP950 vehicle | Glass | 0 | 4.83 | 4.77 | 4.63 | 4.60 | -0.23 |

**Table 5-2: pH values for empty and TRB-loaded AP950 stored at 40°C**

| **Sample** | **Package** | **TRB (wt%)** | **pH value** | | | |
|---|---|---|---|---|---|---|
| | | | **t₀** | **t_{2M}** | **t_{6M}** | **Δt₆₋₀** |
| AP950 | Glass | 2.4 | 4.60 | 4.54 | 4.58 | -0.02 |
| AP950 | Pen appl. | 2.4 | 4.60 | 4.53 | 4.52 | -0.08 |
| AP950 vehicle | Glass | 0 | 4.83 | 4.70 | 4.74 | -0.09 |

For both empty and TRB-loaded systems, the pH decreases upon storage at both 25°C and 40°C, where after storage of 9 months at 25°C and 6 months at 40°C the observed changes in pH values were of *ca.* -0.2 and up to -0.1 respectively. No significant differences were observed between the TRB-loaded system and the vehicle as well as between the different packages.

**Tables 6-1** and **6-2** summarize the assay results (in terms of % of label claim) for AP950 stored at 25°C and 40°C in both glass vials and pen-applicator:

**Table 6-1: TRB assay of AP950 stored at 25°C**

| **Sample** | **Package** | **TRB (wt%)** | **% TRB of label claim** | | | | | |
|---|---|---|---|---|---|---|---|---|
| | | | **t₀** | **t_{2M}** | **t_{3M}** | **t_{6M}** | **t_{9M}** | **Δt**₉₋₀ |
| AP950 | Glass | 2.4 | 94.1 | 97.0 | 100 | 97.9 | 93.9 | -0.2 |
| AP950 | Pen appl. | 2.4 | 94.1 | 93.4 | 95.1 | 93.3 | 90.3 | -3.8 |

**Table 6-2: TRB assay of AP950 stored at 40°C**

| **Sample** | **Package** | **TRB (wt%)** | **% TRB of label claim** | | | | |
|---|---|---|---|---|---|---|---|
| | | | **t₀** | **t_{2M}** | **t_{3M}** | **t_{6M}** | **Δt₆₋₀** |
| AP950 | Glass | 2.4 | 94.1 | 97.8 | 100.7 | 95.4 | 1.3 |
| AP950 | Pen appl. | 2.4 | 94.1 | 87.8 | 86.6 | 85.3 | -8.8 |

As shown in **T**ables 6-1 and 6-2, the levels of **T**RB remain stable at both 25°C and 40°C when the formulation was stored in glass vials. At 25°C only slight decrease (within the measurement error) was detected. Overall, AP950 formulation is stable upon storage under accelerated and standard conditions.

The levels of impurities were also monitored as a part of the stability study. The levels of impurities (in %) are shown in **Tables 7-1** and **7-2.**

**Table 7-1: Impurities levels observed for AP950 stored at 25°C**

| **Sample** | **Package** | **TRB (wt%)** | **% Impurities** | | | | | |
|---|---|---|---|---|---|---|---|---|
| | | | **t₀** | **t_{2M}** | **t_{3M}** | **t_{6M}** | **t_{9M}** | **Δt₉₋₀** |
| AP950 | Glass | 2.4 | 0 | 0.07 | 0.125 | 0.331 | 0.54 | 0.54 |
| AP950 | Pen appl. | 2.4 | 0 | 0.06 | 0.09 | 0.46 | 0.45 | 0.45 |

**Table 7-2: Impurities levels observed for AP950 stored at 40°C**

| **Sample** | **Package** | **TRB (wt%)** | **% Impurities** | | | | |
|---|---|---|---|---|---|---|---|
| | | | **t₀** | **t_{2M}** | **t_{3M}** | **t_{6M}** | **Δt₆₋₀** |
| AP950 | Glass | 2.4 | 0 | 0.04 | 0.13 | 0.80 | 0.80 |
| AP950 | Pen appl. | 2.4 | 0 | 0.15 | 0.09 | 0.57 | 0.57 |

Upon storage at 25°C and 40°C, the levels of impurities gradually increase and reach values of up to 0.8% at 40°C and 0.5% at 25°C. Despite the increase, the detected levels are within the limits commonly defined by the regulatory authorities (NMT 1% of unknown impurities and NMT 2% of total impurities). No significant changes (within the measurement error) in impurities levels were observed between the two package types.

### Performance studies

To evaluate the performance of the formulations in delivering the active compound to skin and nail tissues, a series of experiments was conducted using Franz cells, where the target membranes were either porcine skin (harvested from pig's ears) or human toenails.

### Skin penetration studies

The study was conducted utilizing the following protocol. Receptor chambers of 5mL were filled with the phosphate buffer of pH 7.6. Skin pieces of *ca.* 1.5×1.5cm² were placed onto the receptor chamber orifice (9mm in diameter) and the donor compartment was clamped on top of the skin. Sample of *ca.* 85 mg of the formulation was placed in the donor compartment, directly contacting the skin. The system was sealed and set under controlled temperature to 32±2°C for 24 hours. The liquid from the receptor chamber (referred as 'RC') and the skin piece, after being gently washed from samples residuals (referred as 'Skin'), were collected. TRB levels in each layer (RC and skin) were determined by HPLC.

The penetration results of several independent studies in mg-TRB per surface area and in percentage from applied dose are provided in **Table 8.**

**Table 8: Permeation of TRB into skin and RC layers**

| **Formul.** | **TRB content (wt%)** | **Skin permeation (mg/cm²) [% from applied dose]** | **RC permeation (mg/cm²) [% from applied dose]** |
|---|---|---|---|
| AP950 | 2.4 | 0.264±0.056 [8.99±2.19] | 0.124±0.050 [4.25±1.98] |
| AP3000 | 2.4 | 0.201±0.043 [6.86±1.631] | 0.090±0.049 [3.07±1.86] |
| AP3500 | 2.4 | 0.212±0.034 [10.38±1.93] | 0.055±0.036 [1.89±1.43] |
| AP4500 | 2.4 | 0.388±0.092 [13.25±3.64] | 0.118±0.029 [4.02±1.41] |
| AP_05 | 5.0 | 0.430±0.181 [8.27±1.75] | 0.081±0.034 [1.32±0.59] |
| MUR_5A | 5.0 | 0.605±0.278 [9.92±4.56] | 0.083±0.052 [1.35±0.89] |
| MUR_5B | 5.0 | 0.805±0.404 [14.60±6.67] | 0.023±0.014 [0.38±0.24] |
| MUR_5C | 5.0 | 0.537±0.281 [8.80±4.60] | 0.090±0.045 [1.47±0.77] |

On average, the prototypes loaded with 2.4wt% TRB showed levels of penetrated TRB of *ca.* 6.8-13.3% and 1.9-4.3% in the skin and the RC, respectively. Among all prototypes of 2.4wt% TRB, prototype AP4500 showed the highest levels of penetrated TRB in both skin and RC.

In case of the systems loaded with 5.0% TRB, the absolute penetration of TRB was higher than its penetration of the lower concentration (2.4%) showing *ca.* 0.43-0.81 mg/cm² compared to 0.24 mg/cm² in the skin, and 0.02-0.09 mg/cm² compared to 0.07 mg/cm² in the RC. The accumulation of TRB in the skin was significantly greater for the higher TRB concentration compared to the lower TRB content. However, in the RC it seems that the TRB concentration has much smaller impact on the permeation. In terms of permeation percentage (from applied dose), similar results were observed for the systems of both 2.4% and 5.0% TRB, with one exception of MUR_5B which showed superior penetration of TRB into the skin.

### Nail penetration studies

The study was conducted according to the following protocol. The system was assembled as described for the skin permeation study, however using nails/hooves placed in a dedicated adaptor. Samples of *ca.* 85 mg of tested compositions were placed in the donor compartment, directly contacting the nail/hoof. The system was run for seven days where every 24 hours the nail/hoof was washed with water, and an additional 85 mg of sample were applied on the same area. By the end of the seventh day, each nail/hoof was removed, and washed with distilled water.

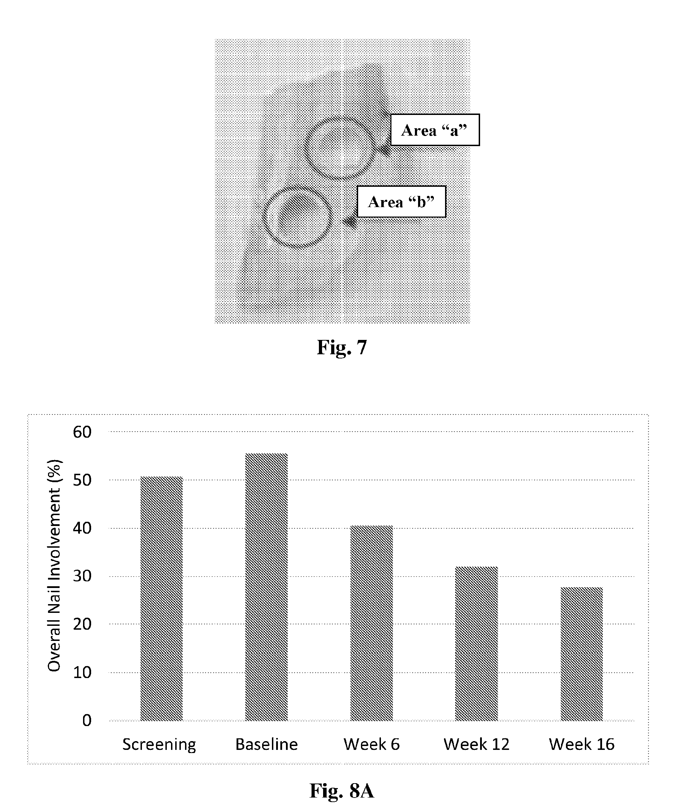

The nail/hoof was drilled at two positions using Dremel 3000: Position a - where the sample was in contact with the nail/hoof, and Position b - an adjacent position where the sample was not in contact with the nail/hoof (as seen in **Fig. 7**). Sample of about 4.0 mg of the drilled nails/hooves were accurately weighed. TRB levels in the nail/hoof were analyzed by HPLC.

In **Table 9,** the accumulated permeation levels of TRB for AP950 system compared to the commercial product of Lamisil^{®} Once are shown, in terms of penetrated TRB per toenail (µgTRB/mgTN) upon continuous exposure for 7 days.

**Table 9: TRB permeation into toenail for AP950 and Lamisil^{®} Once**

| **Formul.** | **TRB content (wt%)** | **Position A (µgTRB/mgTN)** | **Position B (µgTRB/mgTN)** |
|---|---|---|---|
| AP950 | 2.4 | 1.50±1.06 | 1.37±0.73 |
| Lamisil Once | 1.0 | 0.70±0.30 | 0.22±0.07 |

As demonstrated in **Table 9,** the accumulated penetration of TRB when administered in AP950 is 2-6 times higher compared to Lamisil^{®} Once, depending on the examined position. Overall, the results indicate that AP950 formulation is capable of enhancing the penetration of TRB into the nail plate, both vertically and horizontally, better than the commercial product.

The measured mean, minimum and maximum values that were obtained from the experiments above were further used to assess the exposure to TRB following a week of treatment with the prototype AP950 loaded with 2.4wt% TRB-HCl.

For exposure calculation the following data was used: contact area - 6.165 mm²; applied sample - 4.0 mg; minimal mail dimensions - 234.5 mm²; maximal nail dimensions - 441.8 mm². For nail dimension calculations a rectangular shape was assumed. Toenail lengths were determined as reported by Johnson and Shuster [7], where the minimal and maximal reported lengths were 14.6 mm and 18.8 mm respectively. Toenail width was assumed to vary with respect to toenail length where Wₘᵢₙ=1.1Lₘᵢₙ and Wₘₐₓ=1.25Lₘₐₓ.

**Table 10** summarizes the evaluated exposure to TRB-HCl obtained for one week of daily use (once a day) of AP950 system loaded with 2.4wt% TRB.

**Table 10: Exposure evaluation to TRB-HCl**

| | **Nail size** | **Based on permeated TRB as measured in FC** | | | | **Nail surface area (mm²)** | **Total TRB permeation into nail (µg)** |
|---|---|---|---|---|---|---|---|
| | | **TRB/Nail (µg/mg)** | **Nail weight (mg)** | **Nail contact area (mm²)** | **TRB/Sₙₐᵢₗ (µg/mm²)** | | |
| **Mean** | s | 1.50 | 4.0 | 6.158 | 0.31 | 234.5 | 154.7 |
| | XL | 1.50 | 4.0 | 6.158 | 0.31 | 441.8 | 71.8 |
| **Min** | s | 0.65 | 4.0 | 6.158 | 0.13 | 234.5 | 66.8 |
| | XL | 0.65 | 4.0 | 6.158 | 0.13 | 441.8 | 31.0 |
| **Max** | s | 3.77 | 4.0 | 6.158 | 0.77 | 234.5 | 387.7 |
| | XL | 3.77 | 4.0 | 6.158 | 0.77 | 441.8 | 180.0 |

As demonstrated in **Table 10,** the exposure to TRB is expected to lay within the range of about 30-390 µg, depending on toenail's dimensions. The MIC₅₀ (minimum inhibitory concentration required for 50% inhibition) of TRB is evaluated by 0.03-0.5 µg/mL for both *Trichophyton rubrum* and *Trichophyton mentagrophytes* [8,9]. Based on the results presented here, the estimated TRB exposure achieved by applying AP950 reaches the required levels to accomplish effective treatment.

In additional experiments, the penetration of TRB to hoof membrane was examined. As a keratinized tissue, hooves can serve as an alternative model for nails. The permeation of TRB into a horse hoof was tested for formulation AP4500. The results in terms of µgTRB/mgHOOF are presented in the **Table 11.** Note that drilling was carried out at the application area (referred as position A) where the samples were in contact with the hoof.

**Table 11: TRB permeation into hooves**

| **Composition** | **TRB content (wt%)** | **Mean permeation (µgTRB/mgTN)** | **Max. permeation (µgTRB/mgTN)** | **Min. permeation (µgTRB/mgTN)** |
|---|---|---|---|---|
| AP4500 | 2.4 | 1.56±1.00 | 0.40 | 2.94 |

As can be seen, following seven days the accumulated permeation of TRB into the hooves was 1.56 µg/mg, similarly to its penetration into the nail (when being formulated in AP950).

### Phase I/IIA clinical study for the treatment of onychomycosis

Onychomycosis (*tinea unguium*) is a chronic and common fungal infection of the nail, leading to nail discoloration, thickening, and possible separation from the underlying nailbed. Toenails susceptible to infection are higher due to their slower growth, lacking effective source of immune cells, repeated possible cycles of reinfection and ideal fungal growth environment, such as dark and moist. Onychomycosis may also affect the quality of life since its physical appearance can cause anxiety, distress, embarrassment, low self-esteem, and social withdrawal. Onychomycosis is divided into several classes with the main one being Distal and Lateral Subungual Onychomycosis (DLSO) which is the most common one and therefore, was the disease of choice to be treated.

### Study protocol

A Phase I/IIa, open label, single center, exploratory study to assess primarily the safety and secondary the efficacy of a topical Test Formulation according to this disclosure (2.4 wt% terbinafine hydrochloride) administered by pen applicator twice a day, for the treatment of patients with mild to moderate Onychomycosis (great toenail fungi infection).

Subjects initiated the study received the Test Formulation for the total treatment of either 16-24 weeks, to be applied twice a day (evening and morning) on the targeted great toenail (TGT) and the surrounding skin, after cleaning and drying the treated area. The subjects were monitored for any Adverse Event (AE), Serious Adverse Event (SAE) and/or Local Tolerability Reactions (LTR). In addition, the TGT onychomycosis was assessed (% of infected nail) and the mm of healthy nail growth was measured, the involved nail was marked with a permanent marker and photographed to evaluate and monitor the healthy nail growth. At weeks 16 subjects' onychomycosis status was evaluated, and if the healthy nail was found to be greater or equal to 4mm it was considered end of treatment (EOT); if the healthy clear nail was smaller than 4mm, the subject was asked to continue treatment for additional 8 weeks. Samples of the TGT were taken using a scalpel, at both baseline visit (before initiation of treatment) and at the EOT visit, and sent to a microbiology lab for testing the presence or absence of fungi by a KOH wet-mount microscopy test and the type of the fungi by placing the sample on a culture specific plate.

### Safety results

Most of the subjects (67%) had completed treatment after 16 weeks, and only the minority of subjects (33%) had to continue to 24 weeks of treatment. In addition, the dropout rate was relatively low (12%), demonstrating that treatment was relatively easy to execute.

No adverse effects were reported as a result of the treatment. Thus, the Test Formulation was found to be well tolerated, and safe for topical use. No local tolerability reaction, or any related or non- related adverse event or reaction, as well as no serious adverse events or reactions were recorded during the study for any of the subjects.

### Clinical efficacy rate results

Three independent tests were conducted to establish the efficacy-rate of the tested drug for the treatment of onychomycosis including:
(1) The change in lesion free area measured by calculating the delta of healthy nail at the end of the study compared to that measured at bassline treatment [EOT-Baseline=Δ healthy nail in mm];
(2) KOH microscopy test (Negative / Positive); and
(3) Culture (Negative / Positive).

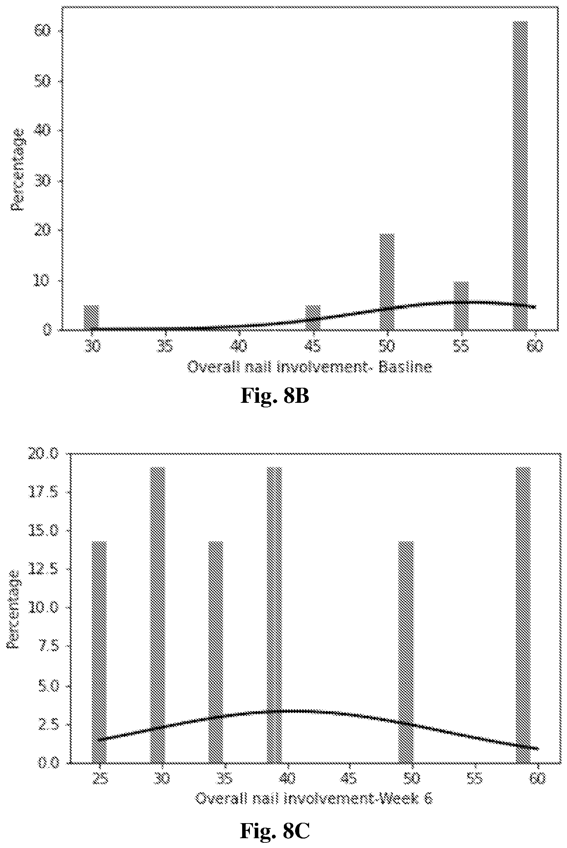

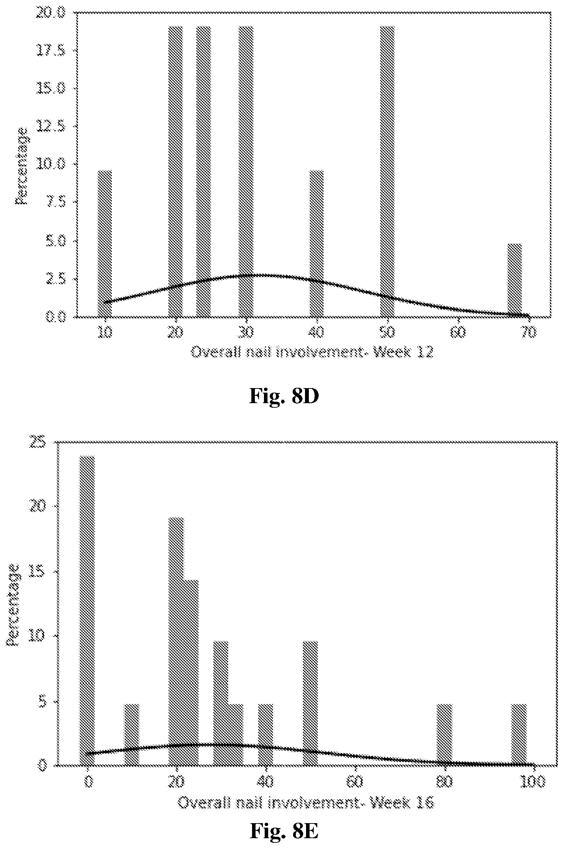

All subjects tested were found to be KOH negative, as well as negative in their culture tests (no fungi growth). The average of healthy nails from all subjects increased from 5 (±1.41) mm measured at baseline to 10 (±4.54) mm at end of treatment. A visible increase in clear/heathy nail from baseline was measured in the majority of subjects, with equal or greater 4mm healthy nail growth, demonstrating clinical efficacy rate of 76.2%, as detailed in **Tables 12-1** and **12-2** and shown in **Figs. 8A-8E****.**

**Table 12-1: Treatment efficacy of the Test Formulation**

| **Parameter** | **Mean (±SD)** | **Median** |
|---|---|---|
| Healthy nail at Baseline (mm) | 5.0 (±1.41) | 5.0 |
| Healthy nail at EOT (mm) | 10.24 (±4.54) | 12.0 |

**Table 12-2: Treatment efficacy of the Test Formulation**

| **Parameter** | **%** |
|---|---|
| Subject showing increase in nail growth greater or equal to 4mm | 76.2 |
| Negative KOH | 100.0 |
| Negative Culture | 100.0 |

**Table 12-3: Average of Overall Nail Involvement (%) during visits**

| **Visit** | **Overall Nail Involvement (%)** |
|---|---|
| Screening | 50.71 |
| Baseline | 55.48 |
| Week 6 | 40.48 |
| Week 12 | 31.9 |
| Week 16 | 27.62 |

### Clinical effectiveness results

Exploratory effectiveness included:
(1) Percentage of nail involvement equal or greater than 10% of the TGT;
(2) Negative KOH; and
(3) Negative culture.

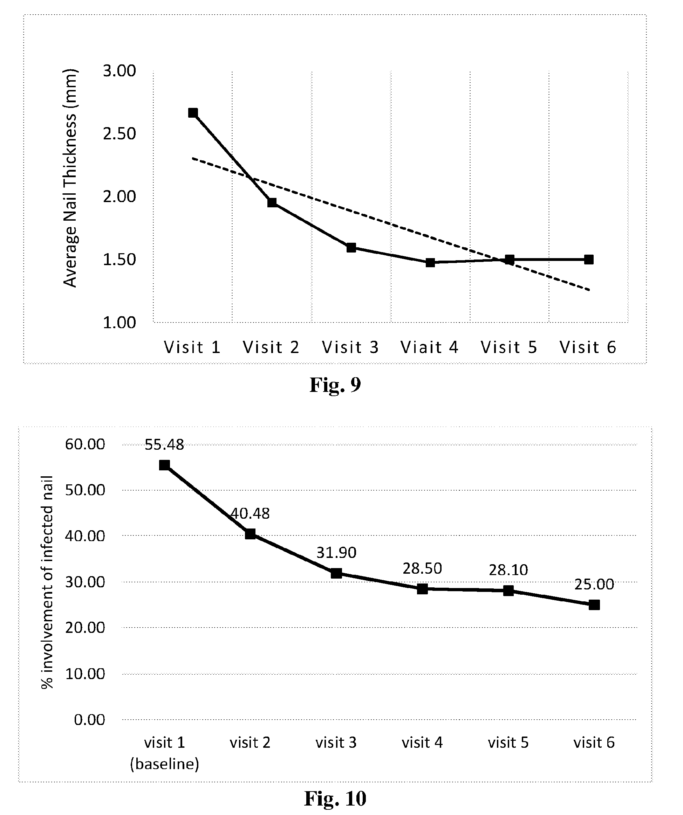

The clinical effectiveness based on all subjects tested was *ca.* 22%, based on subjects that targeted the three parameters listed above. In general, a visible decrease in average percentage of overall infected nail was observed from 55% at baseline to 25% at follow-up visit, as detailed in **Fig. 10** and **Tables 13-1** and **13-2.**

**Table 13-1: Treatment efficacy of the Test Formulation**

| **Parameter** | **Range** | **Mean (±SD)** | **Median** |
|---|---|---|---|
| Involvement of infected nail at baseline (%) | 30 - 60 | 55.48 (±9.52) | 60 |
| Involvement of infected nail at FU visit (%) | 0-60 | 25.0 (±18.91) | 25 |

**Table 13-2: Treatment efficacy of the Test Formulation**

| **Parameter** | **%** |
|---|---|
| Subject with nail involvement ≤10% of the TGT | 22.22 |
| Negative KOH | 100.0 |
| Negative Culture | 100.0 |

### Complete cure

Complete cure is defined as 0% infected nail (complete healthy nail) and negative KOH and negative culture. Three out of the 18 subjects that were tested were found to be completely cured (*ca.* 22%).

### Nail thickness and morphology

Normal nail thickness ranges between 1.0 to 1.5 mm. Nail thickness was evaluated during the study, since it is known to be associated with onychomycosis. The nail thickness usually increases when the nail is infected, and reduces when it is cured.

The average nail thickness of all subjects tested had decreased within the study (**Fig. 9**), indicating nail reverting back to normal thickness and healthy nail.

All the infected nails were dark to brown in color with, in most cases, also some 'broken ends' of the toenails. In all the cases that showed minimum 4 mm growth the yellow and opaque color of the infected nail, changed into smooth, and less yellowish or completely transparent and clear appearance.

### Comparison to commercial products

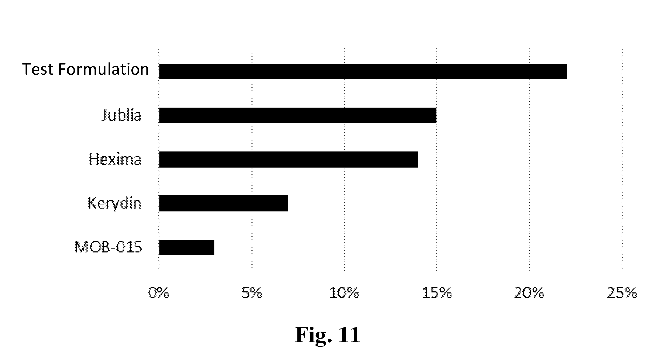

The complete cure rate for the Test Formulation at 16 or 24 weeks was compared to the rate found for the following products Jublia^{®} (efinaconazole), Hexima^{™} (pezadeftide), Kerydin^{™} (lavaborole) and MOB-015 (terbinafine formulation under development of Mobera pharma) after 12 weeks of treatment, and found to be significantly higher, as seen in **Fig. 11****.**

## Claims

1. A formulation for topical delivery of an antifungal active agent to a keratinous tissue, the formulation comprising nanodomains dispersed in an aqueous continuous phase,
the nanodomains comprising said antifungal active agent, at least one hydrophilic surfactant, at least one alcohol, and at least one polyol,
the aqueous phase comprising water, at least one film-forming agent in an amount of no more than about 1 wt%, and at least one keratolytic agent,
wherein the formulation comprises:
a total amount of water of between about 35 wt% and about 55 wt%,
a total amount of hydrophilic surfactants of between about 8 wt% and about 25 wt%,
a total amount of alcohols of between about 12 wt% and about 30 wt%, and
a total amount of polyols of between about 2 wt% and about 12 wt%,
wherein the weight ratio between the total amount of hydrophilic surfactants and the total amounts of alcohols in the formulation ranges between about 1:1 and about 1:3, and
wherein said antifungal agent is terbinafine or any pharmaceutically acceptable salt, or hydrate thereof, wherein the term about is meant to encompass deviation of ±10% from the specifically mentioned value of a parameter.

2. The formulation of claim 1, being formulated to form a film with the nanodomains embedded therein, once applied onto said keratinous tissue, preferably wherein said film is substantially transparent.

3. The formulation of claim 2, wherein said film comprises no more than about 2 wt% film-forming agent.

4. The formulation of any one of claims 1 to 3, comprising at least one oil in an amount of at most about 1 wt%, preferably wherein the formulation is devoid of oil.

5. The formulation of any one of claims 1 to 4, wherein said at least one hydrophilic surfactant is selected from ethoxylated fatty alcohols, polysorbates, ethoxylated fatty acids, ethoxylated hydrogenated castor oil, ethoxylated non-hydrogenated castor oil ethoxylated glycerides of fatty acids, PEGylated octylphenyl ethers, sugar esters, polyglycerol esters, polyethylene glycol (PEG), copolymers of ethylene oxide (EO) and propylene glycol (PG) moieties, lysolecithins, and combinations thereof, optionally wherein the formulation comprises at least two hydrophilic surfactants.

6. The formulation of any one of claims 1 to 5, comprising at least one first hydrophilic surfactant being a polysorbate or ethoxylated castor oil, and at least one second hydrophilic surfactant being ethoxylated fatty alcohols, ethoxylated fatty acids, or ethoxylated glycerides of fatty acids.

7. The formulation of any one of claims 1 to 6, wherein said at least one alcohol is selected from ethanol, propanol, isopropanol, butanol, benzyl alcohol and combination thereof, optionally wherein the formulation comprises at least two alcohols.

8. The formulation of any one of claims 1 to 7, wherein said film forming agent is selected from cellulose ethers, polyvinyl alcohol, polyquaternium-10, guar gum, hydroxypropyl guar gum, xanthan gum, gellans, gelatin and gelatin derivatives, Aloe vera gel, amla, carrageenan, oat flour, starch and modified starch, gelatin, ghatty gum, gum Arabic, inulin, Konjac gum, locust bean gum (LBG), fenugreek, marshmallow root, pectin and modified pectin, quinoa extract, red alga, solagum, tragacanth gum (TG), acrylic acid / ethyl acrylate copolymers, carboxyvinyl polymers, polyalkenyl polyether cross-linked acrylic acid polymer, hydrophobically modified crosslinked acrylic acid polymer, and any mixtures thereof.

9. The formulation of any one of claims 1 to 8, wherein said antifungal active agent is present in the formulation in an amount of at least about 1 wt%, optionally wherein said antifungal active agent is present in the formulation in an amount ranging between about 1 wt% and about 10 wt%.

10. The formulation of any one of claims 1 to 9, wherein the formulation comprises at least 10 wt% of volatile components.

11. The formulation of any one of claims 1 to 10, formulated to form a depot of the antifungal active agent over said keratinous tissue,
wherein the formulation being formulated to form a film with the nanodomains embedded therein, once applied onto said keratinous tissue, the film having a residence time on the keratinous tissue of between about 1 hour and 24 hours.

12. A kit for preparing a formulation for topical delivery of an antifungal active agent to a keratinous tissue, the kit comprising:
a first container holding a concentrate that comprises said antifungal active agent, at least one hydrophilic surfactant, at least one alcohol, and at least one polyol;
a second container holding an aqueous solution comprising water, at least one film-forming agent in an amount of no more than about 1 wt% of the total formulation, and at least one keratolytic agent;
means of mixing the concentrate with the aqueous solution to obtain a topical formulation in the form of nanodomains dispersed in an aqueous continuous phase;
the kit being configured to dose the concentrate and the aqueous solution such that:
the total amount of water in the formulation is between about 35 wt% and about 55 wt%,
the total amount of hydrophilic surfactants in the formulation is between about 8 wt% and about 25 wt%,
the total amount of alcohols in the formulation is between about 12 wt% and about 30 wt%, and
the total amount of polyols in the formulation is between about 2 wt% and about 12 wt%,
the weight ratio between the total amount of hydrophilic surfactants and the total amounts of alcohols in the formulation ranges between about 1:1 and about 1:3,
and wherein said antifungal agent is terbinafine or any pharmaceutically acceptable salt, or hydrate thereof, wherein the term about is meant to encompass deviation of ±10% from the specifically mentioned value of a parameter.

13. An applicator for topically applying a formulation of any one of claims 1 to 11 to a keratinous tissue, the applicator comprising at least one compartment holding said formulation and means of application of the formulation onto the keratinous tissue.

14. An applicator for topically applying a formulation of any one of claims 1 to 11 to a keratinous tissue, the applicator comprising:
a first container holding a concentrate that comprises said antifungal active agent, at least one hydrophilic surfactant, at least one alcohol, and at least one polyol;
a second container holding an aqueous solution comprising water, at least one film-forming agent in an amount of no more than about 1 wt% of the total formulation, and at least one keratolytic agent;
means of mixing the concentrate with the aqueous solution to obtain said formulation in the form of nanodomains dispersed in an aqueous continuous phase; and
means of application of the formulation onto the keratinous tissue;
the applicator being configured to dose the concentrate and the aqueous solution such that:
the total amount of water in the formulation is between about 35 wt% and about 55 wt%,
the total amount of hydrophilic surfactants in the formulation is between about 8 wt% and about 25 wt%,
the total amount of alcohols in the formulation is between about 12 wt% and about 30 wt%, and
the total amount of polyols in the formulation is between about 2 wt% and about 12 wt%,
the weight ratio between the total amount of hydrophilic surfactants and the total amounts of alcohols in the formulation ranges between about 1:1 and about 1:3,
and wherein said antifungal agent is terbinafine or any pharmaceutically acceptable salt, or hydrate thereof.

15. A formulation according to any one of claims 1 to 11 for use in treating a fungal infection in a keratinous tissue, optionally wherein said fungal infection is *Tinea unguium* infection.

## Patentansprüche

1. Eine Formulierung zur topischen Verabreichung eines antifungalen Wirkstoffs auf ein Keratingewebe, wobei die Formulierung in einer wässrigen kontinuierlichen Phase dispergierte Nanodomänen umfasst,
wobei die Nanodomänen den antifungalen Wirkstoff, mindestens ein hydrophiles oberflächenaktives Mittel, mindestens einen Alkohol und mindestens ein Polyol umfassen,
wobei die wässrige Phase Wasser, mindestens ein filmbildendes Mittel in einer Menge von nicht mehr als etwa 1 Gew.-% und mindestens ein keratolytisches Mittel umfasst,
wobei die Formulierung umfasst:
eine Gesamtmenge an Wasser zwischen etwa 35 Gew.-% und etwa 55 Gew.-%,
eine Gesamtmenge an hydrophilen oberflächenaktiven Mitteln zwischen etwa 8 Gew.-% und etwa 25 Gew.-%,
eine Gesamtmenge an Alkoholen zwischen etwa 12 Gew.-% und etwa 30 Gew.-% und eine Gesamtmenge an Polyolen zwischen etwa 2 Gew.-% und etwa 12 Gew.-%,
wobei das Gewichtsverhältnis zwischen der Gesamtmenge an hydrophilen oberflächenaktiven Mitteln und der Gesamtmenge an Alkoholen in der Formulierung zwischen etwa 1:1 und etwa 1:3 liegt, und
wobei der antifungale Wirkstoff Terbinafin oder ein pharmazeutisch verträgliches Salz oder Hydrat davon ist, wobei der Begriff "etwa" eine Abweichung von ±10 % vom ausdrücklich genannten Parameterwert abdeckt.

2. Die Formulierung gemäß Anspruch 1, die so formuliert ist, dass sie nach dem Auftragen auf das Keratingewebe einen Film bildet, in den die Nanodomänen eingebettet sind, wobei der Film vorzugsweise im Wesentlichen transparent ist.

3. Die Formulierung gemäß Anspruch 2, wobei der Film nicht mehr als etwa 2 Gew.-% filmbildendes Mittel umfasst.

4. Die Formulierung gemäß einem der Ansprüche 1 bis 3, welche mindestens ein Öl in einer Menge von höchstens etwa 1 Gew.-% umfasst, wobei die Formulierung vorzugsweise frei von Öl ist.

5. Die Formulierung gemäß einem der Ansprüche 1 bis 4, wobei das mindestens eine hydrophile oberflächenaktive Mittel ausgewählt ist aus ethoxylierten Fettalkoholen, Polysorbaten, ethoxylierten Fettsäuren, einem ethoxylierten hydrierten Rizinusöl, einem ethoxyliertem nicht hydrierten Rizinusöl, ethoxylierten Glyceriden von Fettsäuren, PEGylierten Octylphenylethern, Zuckerestern, Polyglycerinestern, Polyethylenglykol (PEG), Copolymeren aus Ethylenoxid (EO)- und Propylenglykol (PG)-Einheiten, Lysolecithinen und Kombinationen davon, wobei die Formulierung gegebenenfalls mindestens zwei hydrophile oberflächenaktive Mittel umfasst.

6. Die Formulierung gemäß einem der Ansprüche 1 bis 5, umfassend mindestens ein erstes hydrophiles oberflächenaktives Mittel, das ein Polysorbat oder ethoxyliertes Rizinusöl ist, und mindestens ein zweites hydrophiles oberflächenaktives Mittel, bei welchem es sich um ethoxylierte Fettalkohole, ethoxylierte Fettsäuren oder ethoxylierte Fettsäureglyceride handelt.

7. Die Formulierung gemäß einem der Ansprüche 1 bis 6, wobei der mindestens eine Alkohol ausgewählt ist aus Ethanol, Propanol, Isopropanol, Butanol, Benzylalkohol und Kombinationen davon, wobei die Formulierung gegebenenfalls mindestens zwei Alkohole umfasst.

8. Die Formulierung gemäß einem der Ansprüche 1 bis 7, wobei das filmbildende Mittel ausgewählt ist aus Celluloseethern, Polyvinylalkohol, Polyquaternium-10, Guarkernmehl, Hydroxypropylguarkernmehl, Xanthan, Gellan, Gelatine und Gelatinederivaten, Aloe-Vera-Gel, Amla, Carrageen, Hafermehl, Stärke und modifizierter Stärke, Gelatine, Ghatti-Gummi, Gummi arabicum, Inulin, Konjakgummi, Johannisbrotkernmehl (LBG), Bockshornklee, Eibischwurzel, Pektin und modifiziertem Pektin, Quinoa-Extrakt, Rotalge, Solagum, Tragantgummi (TG), Acrylsäure-/Ethylacrylat-Copolymeren, Carboxyvinylpolymeren, einem Polyalkenylpolyethervernetzten Acrylsäurepolymer, einem hydrophob modifizierten vernetzten Acrylsäurepolymer sowie Gemischen davon.

9. Die Formulierung gemäß einem der Ansprüche 1 bis 8, wobei der antifungale Wirkstoff in der Formulierung in einer Menge von mindestens etwa 1 Gew.-% vorliegt, wobei der antifungale Wirkstoff in der Formulierung gegebenenfalls in einer Menge im Bereich zwischen etwa 1 Gew.-% und etwa 10 Gew.-% vorliegt.

10. Die Formulierung gemäß einem der Ansprüche 1 bis 9, wobei die Formulierung mindestens 10 Gew.-% flüchtige Bestandteile umfasst.

11. Die Formulierung gemäß einem der Ansprüche 1 bis 10, die so formuliert ist, dass sie ein Depot des antifungalen Wirkstoffs über dem Keratingewebe bildet,
wobei die Formulierung so formuliert ist, dass sie einen Film mit den darin eingebetteten Nanodomänen bildet, und wobei der Film nach dem Auftragen auf das Keratingewebe eine Verweildauer auf dem Keratingewebe von zwischen etwa 1 Stunde und 24 Stunden aufweist.

12. Ein Kit zur Herstellung einer Formulierung zur topischen Verabreichung eines antifungalen Wirkstoffs auf ein Keratingewebe, wobei das Kit umfasst:
einen ersten Behälter, welcher ein Konzentrat enthält, das der antifungale Wirkstoff, mindestens ein hydrophiles oberflächenaktives Mittel, mindestens einen Alkohol und mindestens ein Polyol umfasst;
einen zweiten Behälter, welcher eine wässrige Lösung enthält, die Wasser, mindestens ein filmbildendes Mittel in einer Menge von nicht mehr als etwa 1 Gew.-% der Gesamtformulierung und mindestens ein keratolytisches Mittel umfasst;
Mittel zum Mischen des Konzentrats mit der wässrigen Lösung, um eine topische Formulierung in Form von Nanodomänen zu erhalten, welche in einer wässrigen kontinuierlichen Phase dispergiert sind;
wobei das Kit so konfiguriert ist, dass das Konzentrat und die wässrige Lösung so dosiert werden, dass:
die Gesamtmenge an Wasser in der Formulierung zwischen etwa 35 Gew.-% und etwa 55 Gew.-% liegt,
die Gesamtmenge an hydrophilen oberflächenaktiven Mitteln in der Formulierung zwischen etwa 8 Gew.-% und etwa 25 Gew.-% liegt,
die Gesamtmenge an Alkoholen in der Formulierung zwischen etwa 12 Gew.-% und etwa 30 Gew.-% liegt und
die Gesamtmenge an Polyolen in der Formulierung zwischen etwa 2 Gew.-% und etwa 12 Gew.-% liegt,
das Gewichtsverhältnis zwischen der Gesamtmenge an hydrophilen oberflächenaktiven Mitteln und der Gesamtmenge an Alkoholen in der Formulierung im Bereich zwischen etwa 1:1 und etwa 1:3 liegt,
und wobei der antifungale Wirkstoff Terbinafin oder ein pharmazeutisch verträgliches Salz oder Hydrat davon ist, wobei der Begriff "etwa" eine Abweichung von ±10 % vom ausdrücklich genannten Parameterwert abdeckt.

13. Ein Applikator zum topischen Auftragen einer Formulierung gemäß einem der Ansprüche 1 bis 11 auf ein Keratingewebe, wobei der Applikator mindestens eine Kammer zur Aufnahme der Formulierung und Mittel zum Auftragen der Formulierung auf das Keratingewebe umfasst.

14. Ein Applikator zum topischen Auftragen einer Formulierung gemäß einem der Ansprüche 1 bis 11 auf ein Keratingewebe, wobei der Applikator umfasst:
einen ersten Behälter, der ein Konzentrat enthält, das den antifungalen Wirkstoff, mindestens ein hydrophiles oberflächenaktives Mittel, mindestens einen Alkohol und mindestens ein Polyol umfasst;
einen zweiten Behälter, der eine wässrige Lösung enthält, die Wasser, mindestens ein filmbildendes Mittel in einer Menge von nicht mehr als etwa 1 Gew.-% der Gesamtformulierung und mindestens ein keratolytisches Mittel umfasst;
Mittel zum Mischen des Konzentrats mit der wässrigen Lösung, um die Formulierung in Form von in einer wässrigen kontinuierlichen Phase dispergierten Nanodomänen zu erhalten; und
Mittel zum Auftragen der Formulierung auf das Keratingewebe;
wobei der Applikator so konfiguriert ist, dass das Konzentrat und die wässrige Lösung so dosiert werden, dass:
die Gesamtmenge an Wasser in der Formulierung zwischen etwa 35 Gew.-% und etwa 55 Gew.-% liegt,
die Gesamtmenge an hydrophilen oberflächenaktiven Mitteln in der Formulierung zwischen etwa 8 Gew.-% und etwa 25 Gew.-% liegt,
die Gesamtmenge an Alkoholen in der Formulierung zwischen etwa 12 Gew.-% und etwa 30 Gew.-% liegt, und
die Gesamtmenge an Polyolen in der Formulierung zwischen etwa 2 Gew.-% und etwa 12 Gew.-% liegt,
das Gewichtsverhältnis zwischen der Gesamtmenge an hydrophilen oberflächenaktiven Mitteln und der Gesamtmenge an Alkoholen in der Formulierung im Bereich zwischen etwa 1:1 und etwa 1:3 liegt,
und wobei der antifungale Wirkstoff Terbinafin oder ein pharmazeutisch verträgliches Salz oder Hydrat davon ist.

15. Eine Formulierung gemäß einem der Ansprüche 1 bis 11 zur Verwendung bei der Behandlung einer Pilzinfektion in einem Keratingewebe, wobei die Pilzinfektion gegebenenfalls eine *Tinea-unguium*-Infektion ist.

## Revendications

1. Formulation pour l'administration topique d'un agent actif antifongique à un tissu kératineux, la formulation comprenant des nanodomaines dispersés dans une phase continue aqueuse,
les nanodomaines comprenant ledit agent actif antifongique, au moins un tensioactif hydrophile, au moins un alcool, et au moins un polyol,
la phase aqueuse comprenant de l'eau, au moins un agent filmogène en une quantité non supérieure à environ 1 % en poids, et au moins un agent kératolytique,
laquelle formulation comprend :
une quantité totale d'eau comprise entre environ 35 % en poids et environ 55 % en poids,
une quantité totale de tensioactifs hydrophiles comprise entre environ 8 % en poids et environ 25 % en poids,
une quantité totale d'alcools comprise entre environ 12 % en poids et environ 30 % en poids, et
une quantité totale de polyols comprise entre environ 2 % en poids et environ 12 % en poids,
dans laquelle le rapport en poids entre la quantité totale de tensioactifs hydrophiles et la quantité totale d'alcools dans la formulation est situé dans une plage comprise entre environ 1/1 et environ 1/3, et
dans laquelle ledit agent antifongique est la terbinafine ou n'importe quel sel pharmaceutiquement acceptable ou hydrate de celle-ci, dans laquelle le terme "environ" est destiné à englober un écart de ± 10 % par rapport à la valeur spécifiquement mentionnée d'un paramètre.

2. Formulation selon la revendication 1, qui est formulée pour former un film dans lequel sont incorporés les nanodomaines, une fois qu'elle a été appliquée sur ledit tissu kératineux, de préférence dans laquelle ledit film est substantiellement transparent.

3. Formulation selon la revendication 2, dans laquelle ledit film ne comprend pas plus qu'environ 2 % en poids d'agent filmogène.

4. Formulation selon l'une quelconque des revendications 1 à 3, comprenant au moins une huile en une quantité d'au plus environ 1 % en poids, de préférence laquelle formulation est exempte d'huile.

5. Formulation selon l'une quelconque des revendications 1 à 4, dans laquelle ledit au moins un tensioactif hydrophile est choisi parmi les alcools gras éthoxylés, les polysorbates, les acides gras éthoxylés, l'huile de ricin hydrogénée éthoxylée, l'huile de ricin non hydrogénée éthoxylée, les glycérides éthoxylés d'acides gras, les octylphényléthers PEG-ylés, les esters de sucre, les esters de polyglycérol, le polyéthylèneglycol (PEG), les copolymères de motifs d'oxyde d'éthylène (EO) et de propylèneglycol (PG), les lysolécithines, et leurs combinaisons, éventuellement laquelle formulation comprend au moins deux tensioactifs hydrophiles.

6. Formulation selon l'une quelconque des revendications 1 à 5, comprenant au moins un premier tensioactif hydrophile étant un polysorbate ou une huile de ricin éthoxylée, et au moins un deuxième tensioactif hydrophile étant des alcools gras éthoxylés, des acides gras éthoxylés ou des glycérides éthoxylés d'acides gras.

7. Formulation selon l'une quelconque des revendications 1 à 6, dans laquelle ledit au moins un alcool est choisi parmi l'éthanol, le propanol, l'isopropanol, le butanol, l'alcool benzylique et leurs combinaisons, éventuellement laquelle formulation comprend au moins deux alcools.

8. Formulation selon l'une quelconque des revendications 1 à 7, dans laquelle ledit agent filmogène est choisi parmi les éthers de cellulose, le poly(alcool vinylique), le polyquaternium 10, la gomme de guar, la gomme d'hydroxypropyl-guar, la gomme xanthane, les gellanes, la gélatine et les dérivés de gélatine, le gel d'aloe vera, l'amla, la carraghénane, la farine d'avoine, l'amidon et l'amidon modifié, la gélatine, la gomme ghatty, la gomme arabique, l'inuline, la gomme de konjac, la gomme de caroube (LBG), le fenugrec, la racine de guimauve, la pectine et la pectine modifiée, l'extrait de quinoa, les algues rouges, le Solagum, la gomme adragante (TG), les copolymères d'acide acrylique / acrylate d'éthyle, les polymères carboxyvinyliques, les polymères d'acide acrylique réticulés par un polyéther polyalcénylique, les polymères d'acide acrylique réticulé modifiés hydrophobiquement, et l'un quelconque de leurs mélanges.

9. Formulation selon l'une quelconque des revendications 1 à 8, dans laquelle ledit agent actif antifongique est présent dans la formulation en une quantité d'au moins environ 1 % en poids, éventuellement dans laquelle ledit agent actif antifongique est présent dans la formulation en une quantité située dans une plage comprise entre environ 1 % en poids et environ 10 % en poids.

10. Formulation selon l'une quelconque des revendications 1 à 9, dans laquelle la formulation comprend au moins 10 % en poids de composants volatils.

11. Formulation selon l'une quelconque des revendications 1 à 10, formulée pour former un dépôt de l'agent actif antifongique sur ledit tissu kératineux,
dans laquelle la formulation étant formulée pour former un film dans lequel sont incorporés les nanodomaines, une fois qu'elle a été appliquée sur ledit tissu kératineux, le film ayant un temps de séjour sur le tissu kératineux compris entre environ 1 heure et 24 heures.

12. Kit pour préparer une formulation pour l'administration topique d'un agent actif antifongique à un tissu kératineux, le kit comprenant :
un premier récipient contenant un concentré qui comprend ledit agent actif antifongique, au moins un tensioactif hydrophile, au moins un alcool, et au moins un polyol ;
un deuxième récipient contenant une solution aqueuse comprenant de l'eau, au moins un agent filmogène en une quantité non supérieure à environ 1 % en poids de la formulation totale, et au moins un agent kératolytique ;
un moyen pour mélanger le concentré avec la solution aqueuse pour obtenir une formulation topique sous la forme de nanodomaines dispersés dans une phase continue aqueuse ;
le kit étant configuré pour doser le concentré et la solution aqueuse de façon que :
la quantité totale d'eau dans la formulation soit comprise entre environ 35 % en poids et environ 55 % en poids,
la quantité totale de tensioactifs hydrophiles dans la formulation soit comprise entre environ 8 % en poids et environ 25 % en poids,
la quantité totale d'alcools dans la formulation soit comprise entre environ 12 % en poids et environ 30 % en poids, et
la quantité totale de polyols dans la formulation soit comprise entre environ 2 % en poids et environ 12 % en poids,
le rapport en poids entre la quantité totale de tensioactifs hydrophiles et la quantité totale d'alcools dans la formulation soit situé dans une plage comprise entre environ 1/1 et environ 1/3,
et dans lequel ledit agent antifongique est la terbinafine ou n'importe quel sel pharmaceutiquement acceptable ou hydrate de celle-ci, dans lequel le terme "environ" est destiné à englober un écart de ± 10 % par rapport à la valeur spécifiquement mentionnée d'un paramètre.

13. Applicateur pour l'application topique d'une formulation de l'une quelconque des revendications 1 à 11 sur un tissu kératineux, l'applicateur comprenant au moins un compartiment contenant ladite formulation et un moyen pour appliquer la formulation sur le tissu kératineux.

14. Applicateur pour l'application topique d'une formulation de l'une quelconque des revendications 1 à 11 sur un tissu kératineux, l'applicateur comprenant :
un premier récipient contenant un concentré qui comprend ledit agent actif antifongique, au moins un tensioactif hydrophile, au moins un alcool, et au moins un polyol ;
un deuxième récipient contenant une solution aqueuse comprenant de l'eau, au moins un agent filmogène en une quantité non supérieure à environ 1 % en poids de la formulation totale, et au moins un agent kératolytique ;
un moyen pour mélanger le concentré avec la solution aqueuse pour obtenir ladite formulation sous la forme de nanodomaines dispersés dans une phase continue aqueuse ; et
un moyen pour appliquer la formulation sur le tissu kératineux ;
l'applicateur étant configuré pour doser le concentré et la solution aqueuse de façon que :
la quantité totale d'eau dans la formulation soit comprise entre environ 35 % en poids et environ 55 % en poids,
la quantité totale de tensioactifs hydrophiles dans la formulation soit comprise entre environ 8 % en poids et environ 25 % en poids,
la quantité totale d'alcools dans la formulation soit comprise entre environ 12 % en poids et environ 30 % en poids, et
la quantité totale de polyols dans la formulation soit comprise entre environ 2 % en poids et environ 12 % en poids,
le rapport en poids entre la quantité totale de tensioactifs hydrophiles et la quantité totale d'alcools dans la formulation soit situé dans une plage comprise entre environ 1/1 et environ 1/3,
et dans lequel ledit agent antifongique est la terbinafine ou n'importe quel sel pharmaceutiquement acceptable ou hydrate de celle-ci.

15. Formulation selon l'une quelconque des revendications 1 à 11 pour une utilisation dans le traitement d'une infection fongique dans un tissu kératineux, éventuellement dans laquelle ladite infection fongique est une infection par *Tinea unguium.*
